# EUROPEAN PATENT SPECIFICATION

(11) **EP 1 809 315 B1**
(45) Date of publication and mention of the grant of the patent: **01.07.2009**
(21) Application number: 05810019.9
(22) Date of filing: 17.10.2005
(51) Int. Cl.: A61K 38/17, A61P 35/00

(54) **INHIBITION OF TUMOR GROWTH AND METASTASIS BY ATF2-DERIVED PEPTIDES**
HEMMUNG DES TUMORWACHSTUMS UND DER METASTASENBILDUNG MIT VON ATF2 STAMMENDEN PEPTIDEN
INHIBITION DE CROISSANCE TUMORALE ET DE MÉTASTASE À L'AIDE DE PEPTIDES DÉRIVÉS DE ATF-2

(30) Priority: 18.10.2004 US 620184 P
(43) Date of publication of application: 25.07.2007
(73) Proprietor: The Mount Sinai School of Medicine of New York University, New York, NY 10029 (US)
(72) Inventor: BHOUMIK, Anindita, San Diego, CA 92130 (US); RONAI, Ze'ev, La Jolla, CA 92037 (US)
(74) Representative: Olsen, Lars Pallisgaard
(86) International application number: PCT/US2005/037634
(87) International publication number: WO 2006/044984

(56) References cited:
- WO-A-02/064025
- BHOUMIK A ET AL: "Activating transcription factor 2-derived peptides alter resistance of human tumor cell lines to ultraviolet irradiation and chemical treatment" CLINICAL CANCER RESEARCH, THE AMERICAN ASSOCIATION FOR CANCER RESEARCH, US, vol. 7, no. 2, February 2001 (2001-02), pages 331-342, XP002286103 ISSN: 1078-0432
- BHOUMIK A ET AL: "An ATF2-derived peptide sensitizes melanomas to apoptosis and inhibits their growth and metastasis" JOURNAL OF CLINICAL INVESTIGATION, NEW YORK, NY, US, vol. 110, no. 5, September 2002 (2002-09), pages 643-650, XP002286105 ISSN: 0021-9738
- BHOUMIK A ET AL: "Transcriptional switch by activating transcription factor 2-derived peptide sensitizes melanoma cells to apoptosis and inhibits their tumorigenicity" PROCEEDINGS OF THE NATIONAL ACADEMY OF SCIENCES OF USA, NATIONAL ACADEMY OF SCIENCE, WASHINGTON, DC, US, vol. 101, no. 12, 23 March 2004 (2004-03-23), pages 4222-4227, XP002286106 ISSN: 0027-8424

## Description

This application claims priority from U.S. Serial No. 60/620,184, filed October 18, 2005.

### STATEMENT REGARDING FEDERALLY SPONSORED RESEARCH

The research leading to the present invention was supported, in part, by the National Cancer Institute through grant CA 99961. Accordingly, the U.S. government has certain rights in the invention.

### TECHNICAL FIELD

The present disclosure generally relates to treating hyperprolifertive diseases, particularly malignant neoplasms that are resistant to radiation therapy, chemotherapy, or both. In particular, the present disclosure relates to compounds and methods for altering the activity of transcription factor ATF2 to render hyperprolifertive cells sensitive to various therapies by, for example, priming or promoting apoptosis.

### BACKGROUND

The notorious resistance of melanoma to treatment, along with its strong potential to metastasize, represents a major clinical obstacle in the treatment of tumors of this type. A growing body of knowledge points to numerous changes in the apoptosis cascades that take place in such tumors. The effects of these changes are to render such tumors insensitive to apoptosis following a wide range of treatments (reviewed in Soengas and Lowe, Oncogene, 2003; 22:3138-3151; and Ivanov et al., Oncogene, 2003; 22:3152-3161). Among the changes identified is the activation of certain signaling cascades, including MAPK, as a result of an activating mutation within B-Raf and N-Ras (Davies et al., Nature, 2002; 417:949-954; Smalley, Int. J. Cancer, 2003; 104:527-532), resulting in the constitutive activation of downstream effectors, *i*.*e*., stress activated kinases and their respective transcription factors (Hsu et al., Cell 1996; 84:299-308; Liu et al., Cell 1996; 87:565-576; Arch et al., Genes & Dev. 1998; 12:2821-2830). Despite advances in understanding the biology of melanoma (Meier et al., Frontiers in Bioscience 1998, 3: d1005-1010), the nature of its resistance to radiation-induced apoptosis remains largely unknown.

Several transcription factors likely to serve as primary targets of altered signaling cascades, including ATF2, AP2, Jun, STAT3 and NF-κB, have been implicated in melanoma development and progression (Ivanov et al., Mol. Cell, 2001; 7:517-28; Ivanov et al., Oncogene, 2001; 20:2243-53; Ivanov et al., J. Biol. Chem., 2002; 277:4932-44; Bar-Eli, Pigment Cell Research, 2001; 14:78-85; Ronai et al., Oncogene, 1998; 16:523-531). ATF2 is a member of the bZIP family of transcription factors that requires heterodimerization with other members of this family, such as c-Jun, JunD, JunB, Fos, Fra1, ATFx and ATFa (Newman and Keating, Science, 2003, 300:2097-2101; Steinmuller et al., Biochem J., 2001; 360:599-607), and other regulatory components, such as NF-κB and Rb (Kaszubska et al., Mol. Cell Biol., 1993, 13:7180-90; Kim et al., Nature, 1992, 358:331-4), to elicit its transcriptional activities. Also prerequisite for activity is ATF2 phosphorylation by JNK and p38 (Ouwens et al., EMBO J., 2002, 21:3782-3793; Gupta et al., Science, 1995, 267:389-393). Hypophosphorylated or transcriptionally inactive forms of ATF2 elicit a silencing effect on TNF expression, which mediates an anti-apoptotic signal and results in increased apoptosis (Ivanov et al., J. Biol. Chem. 1999; 274:14079-14089). Inhibition of ATF2 activities results in sensitization of melanoma, as well as breast cancer cells, to apoptosis following treatment with radiomimetic or chemotherapeutic drugs that by themselves fail to affect these tumors (Bhoumik et al., Clin. Cancer Res., 2001, 2:331-342).

The following documents disclose amino terminal peptides of ATF2 comprising amino acid residues 50 to 100 of ATF2 for promoting apoptosis and inhibiting the growth of tumour cells; WO 02/064025; Bhoumik et al, Clinical Cancer Research, vol. 7, no. 2, Feb. 2001, pp 331-342; Bhoumik et al, Journal of Clinical Investigation, vol. 110, No. 5, Sep. 2002, pp 643-650 and; Bhoumik et al, Proc. Nat. Acad. Sci. USA, vol. 101, no. 12, March 2004, pp 4222-4227

Given the resistance of many cancers to radiation therapy and chemotherapy, there is a continuous need for the development of novel chemotherapeutics with novel mechanisms of action. The present invention meets such needs, and further provides other related advantages.

### SUMMARY

In one aspect, the present disclosure provides an amino-terminal peptide of ATF2 consisting of amino acids from residue 51 of ATF2 to 60 of ATF2 for use as a pharmaceutical composition.

Also disclosed is a method of sensitizing a tumor cell to apoptosis by contacting a tumor cell with a compound that alters ATF2 activity, wherein the compound comprises an amino-terminal peptide of ATF2 having amino acids from residue 51 of ATF2 to 60 of ATF2. In related embodiments, the ATF2 peptides alter TRE-dependent transcriptional activity, alter the association of JNK and c-Jun, induce or potentiate apoptosis (basal or induced), or any combination thereof. In certain other embodiments, the ATF2 peptides further comprise a translocation peptide sequence, such as penetratin or HIV-TAT, to aid delivery to the interior of cells *in vivo.*

The method has been specifically exemplified in conditions where the tumor cell is a melanoma tumor cell or a breast cancer tumor cell.

The method of inhibiting tumor cell growth or sensitizing a tumor cell to apoptosis may involve further treating the tumor cell with a chemotherapeutic agent, such as a p38 inhibitor, UCN-01, NCS, anisomycin, LY294002, PD98059, AG490, and SB203580. Alternatively, the invention contemplates further treating the tumor cell with radiation.

In another aspect, the invention provides a polypeptide comprising an amino-terminal peptide fragment ATF2, wherein said ATF2 peptide consists of amino acids from residue 51 of ATF2 to 60 of ATF2. In certain embodiments, the ATF2 peptides further comprise a translocation peptide sequence, such as HA-penetratin or HIV-TAT, to aid delivery to the interior of cells *in vivo.* Naturally, the invention provides nucleic acids and expression vectors encoding such polypeptides.

For treatment or sensitization, the invention provides a pharmaceutical composition comprising the ATF2 peptides of the invention and a pharmaceutically acceptable carrier or excipient. Such pharmaceutical compositions can be used in a method of treating a tumor in a subject, as set forth above in connection with inhibiting growth of a tumorcell or sensitizing such a tumor cell to apoptosis.

These and other aspects of the present invention will become evident upon reference to the following detailed description and attached drawings. In addition, various references are set forth herein that describe in more detail certain procedures or compositions.

### BRIEF DESCRIPTION OF THE DRAWINGS

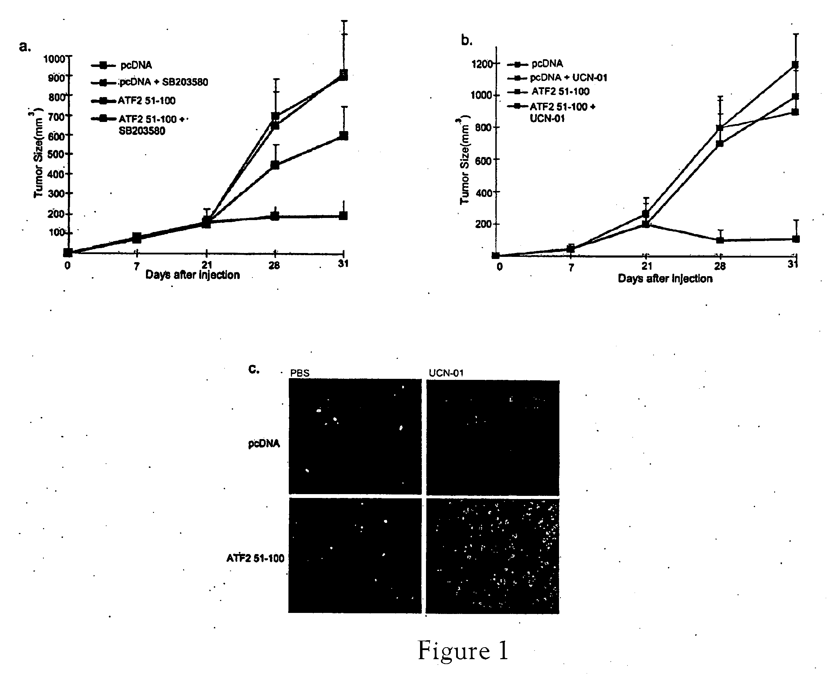

Figures 1A - 1C show ATF2⁵¹⁻¹⁰⁰ peptide inhibits growth of human melanoma cells *in vivo.* LU1205 or FEMX human melanoma tumor-derived cell lines that constitutively express ATF2⁵¹⁻¹⁰⁰ peptide or that contain control vector were each injected subcutaneously into a group of 6 nude mice (10⁶ cells per injection) percell line per experiment. Figure 1A shows data from neo-expressing LU1205 tumors as compared to ATF2⁵¹⁻¹⁰⁰ peptide-expressing LU1205 tumors subjected to the indicated treatment (p38 inhibitor SB203580; 10µM), which was administered three times per week via intra-tumoral injections. The data shown represent three experiments. Bars represent standard deviation, P<0.0015. Figure 1B shows data of growth rate of control FEMX tumor cells as compared to ATF2⁵¹⁻¹⁰⁰ expressing FEMX tumor cells in the presence or absence of UCN-01 (5mg/Kg), which was administered three times per week by gavage. The data shown represent three experiments. Bars represent standard deviation, P< 0.0021. Figure 1C shows histopathological analysis of tumors from Figure 1B to determine the degree of apoptosis using the Tunnel assay.

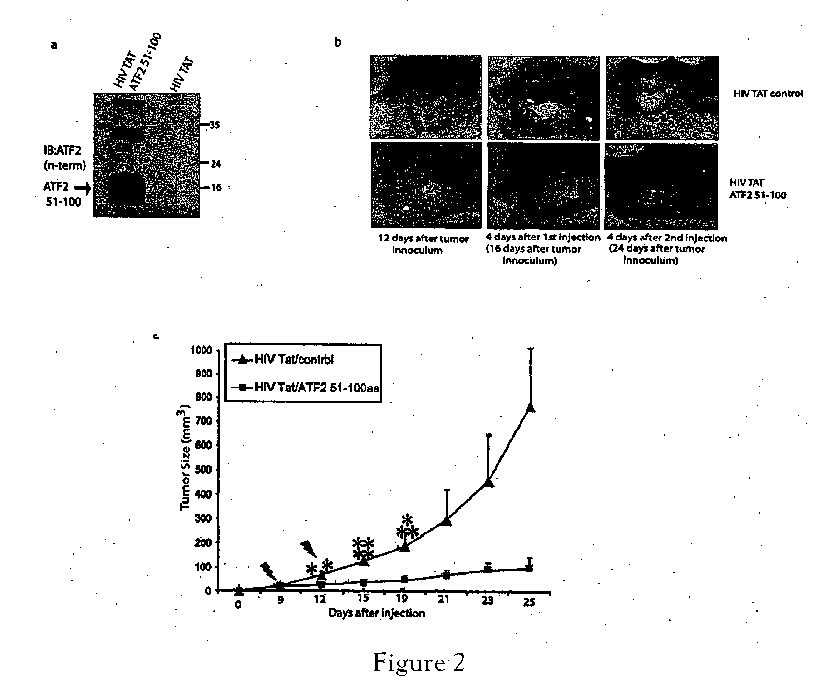

Figures 2A - 2C show the expression of HIV-TAT-ATF2⁵¹⁻¹⁰⁰ peptide inhibits tumorigenicity of SW1 melanoma cells. Figure 2A shows an immunoblot analysis using N-terminal ATF2 antibodies to detect isolated HIV-TAT-fused with ATF2⁵¹⁻¹⁰⁰. Figure 2B shows the assesment of tumor size in C3H mice (SW1 expressing GFP) using UV light illumination at the indicated time points that had been treated with control HIV-TAT alone or HIV-TAT-ATF2⁵¹⁻¹⁰⁰ fusion peptide. Figure 2C shows data obtained from a group of 18 animals studied over the indicated period reflecting changes in the growth of SW1 tumors (P<0.003; T-Test). Arrows point to the time of peptide injection, and stars reflect cases where a tumor was no longer seen (one star = one animal).

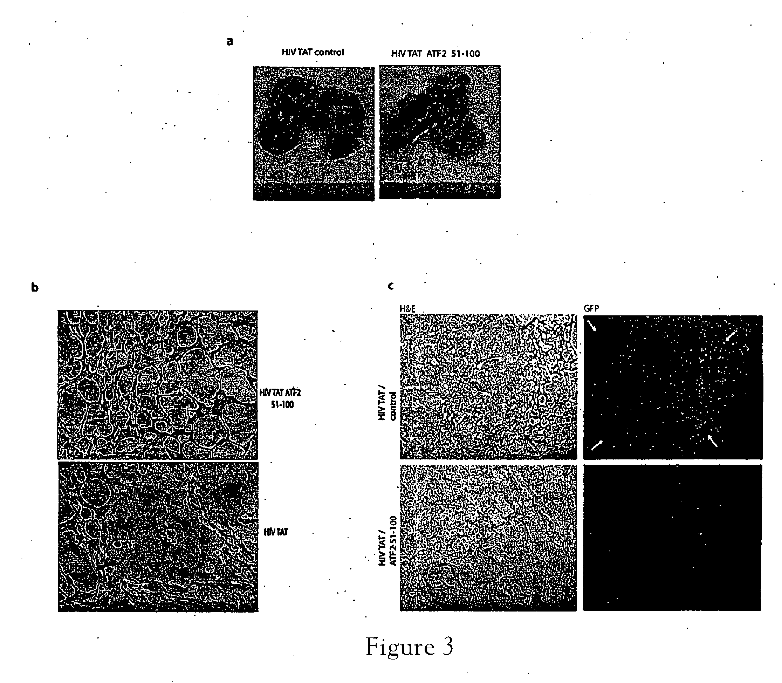

Figures 3A - 3C show that expression of HIV-TAT-ATF2⁵¹⁻¹⁰⁰ peptide inhibits metastasis of SW1 melanoma cells. Analysis of lesions in lungs of animals revealed the presence of metastases in control animals but not those into which the HIV-TAT-ATF2⁵¹⁻¹⁰⁰ peptide had been injected (Figure 3A). Figure 3B shows micrometastasis detected after H&E staining of lungs and liver in the control, but not the HIV-TAT-ATF2⁵¹⁻¹⁰⁰ group. Lung metastases were confirmed by analysis of sections with fluorescence microscopy, which revealed expression of the GFP introduced into the parent SW1 cells prior to their injection (Figure 3C).

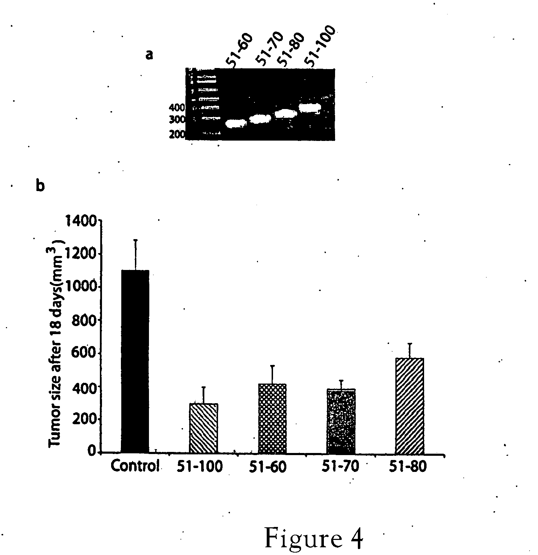

Figures 4A and 4B show that expression of ATF2⁵¹⁻⁶⁰ inhibits melanoma growth *in vivo.* SW1 cells, which constitutively express the peptides indicated in Figure 4A, were analyzed to detect expression of ATF2 peptides at the RNA level using RT-PCR. The indicated MW bands reflect the expected size product. SW1 cells (10⁶) were injected subcutaneously into CH3 mice and tumor growth was monitored for 18 days, at which point tumors were removed and their size carefully assessed (Figure 4B). Each group included 6 animals and each experiment was performed twice (P<0.01; T-test).

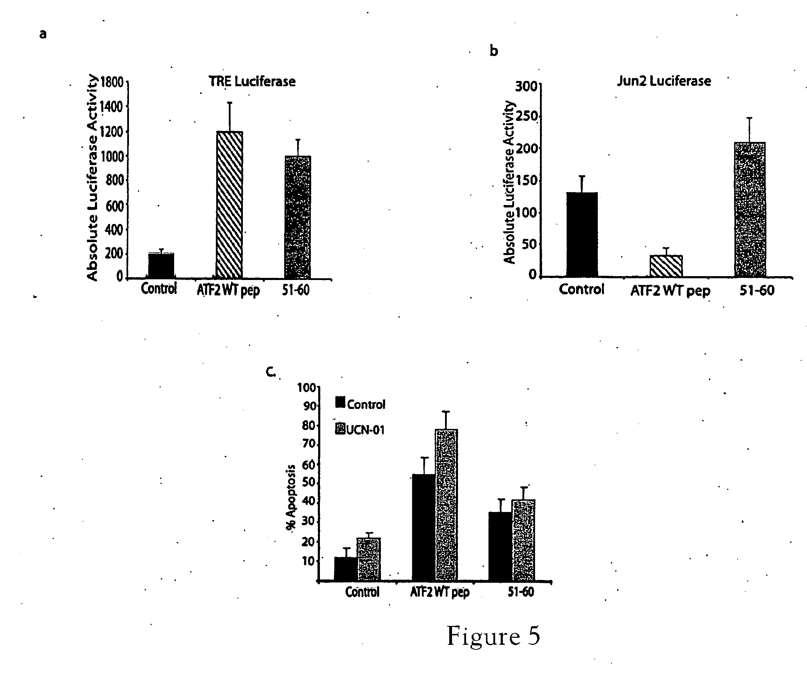

Figures 5A - 5C show that expression of ATF2⁵¹⁻⁶⁰ increases TRE-Luc activity, does not significantly affect Jun2-Luc activity, and induces spontaneous apoptosis of SW1 melanoma cells. SW1 cells constitutively expressing the indicated ATF2 peptides were transfected with either TRE-Luc (Figure 5A) or with Jun2-Luc (Figure 5B), and proteins were prepared for analysis of luciferase activity after 18 hours. Analysis was performed in duplicate, and the data reflect three independent experiments. SW1 cells expressing the ATF2 peptides were analyzed to establish the degree of spontaneous (basal) apoptosis as well as apoptosis 24 hours following treatment with the chemotherapeutic drug UCN-01 (Figure 5C). The data represent triplicate analysis reproduced three times.

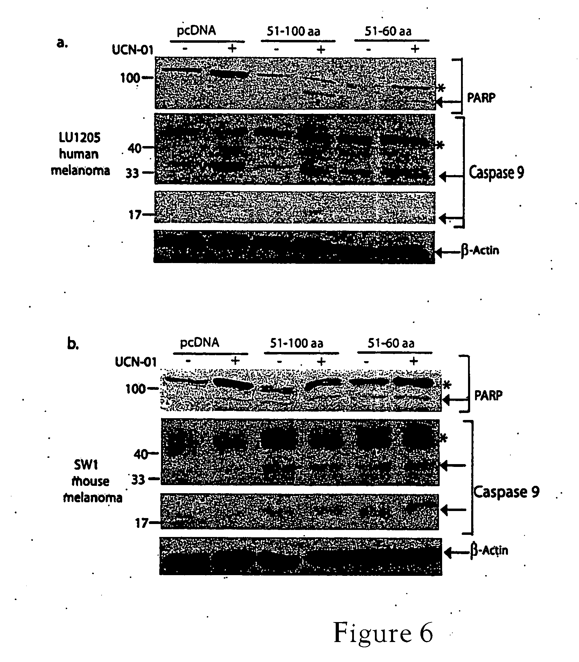

Figures 6A and 6B show that ATF2 peptides induce activation of caspase 9 and PARP cleavage in human and mouse melanoma cells. Human melanoma cells LU1205 were transfected with the nucleic acid molecules that encode either ATF2⁵¹⁻¹⁰⁰ peptide or ATF2⁵¹⁻⁶⁰ peptide. Twenty-four hours after transfection, cells were treated with of UCN-01 (5µm) for 24 hours. Immunoblotting analysis was performed using caspase 9 and PARP antibodies, and β-actin was used as a loading control (Figure 6A). The same experiment was performed in SW-1 cells (Figure 6B). Stars point to the position of the uncleaved form whereas arrows point to the cleaved products.

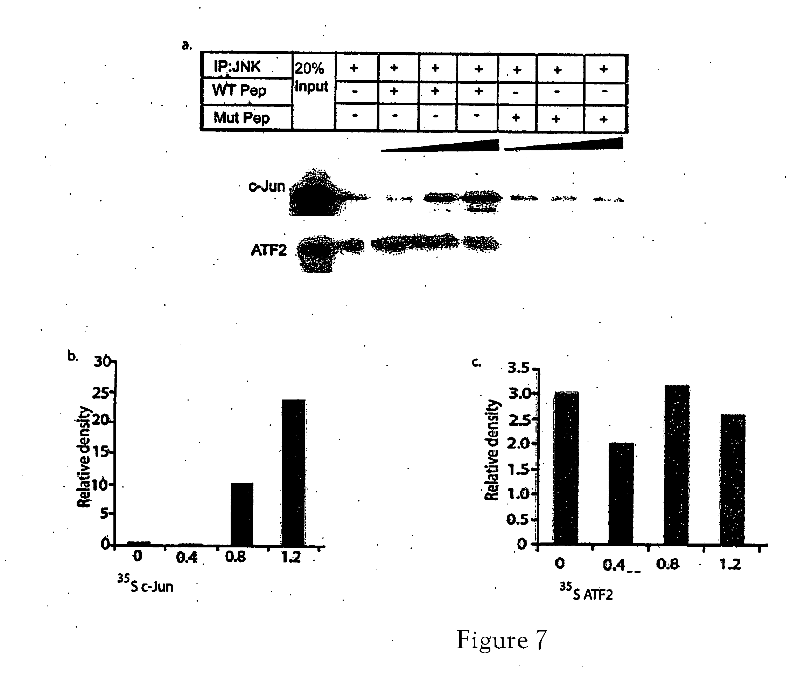

Figures 7A - 7C show that ATF2⁵¹⁻⁶⁰ peptide increases Jun association with JNK. HA-JNK was transfected into 293T cells and 24h later JNK was immunoprecipitated and bound to protein G beads. Bead-bound JNK was incubated with *in vitro*-translated c-Jun or ATF2 in the absence or presence of the wt or mutant form of ATF2⁵¹⁻⁶⁰. Following incubation bead-bound JNK was washed (*see* Example 4) and the amount of bound Jun or ATF2 was assessed *via* SDS-PAGE analysis subjected to autoradiography (Panel A) and quantification with the aid of a phosphor imager (Panels B and C).

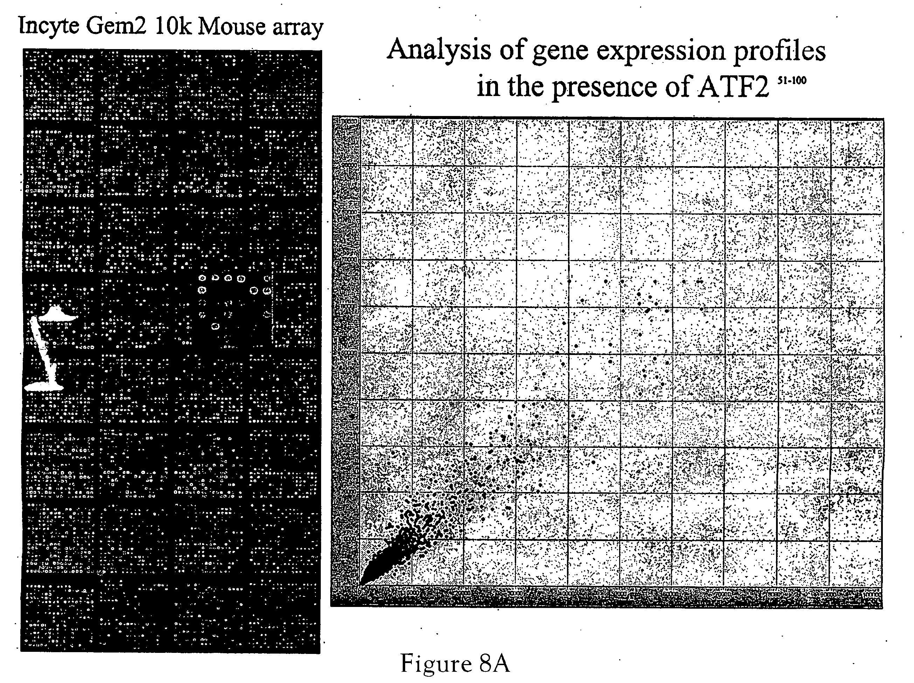

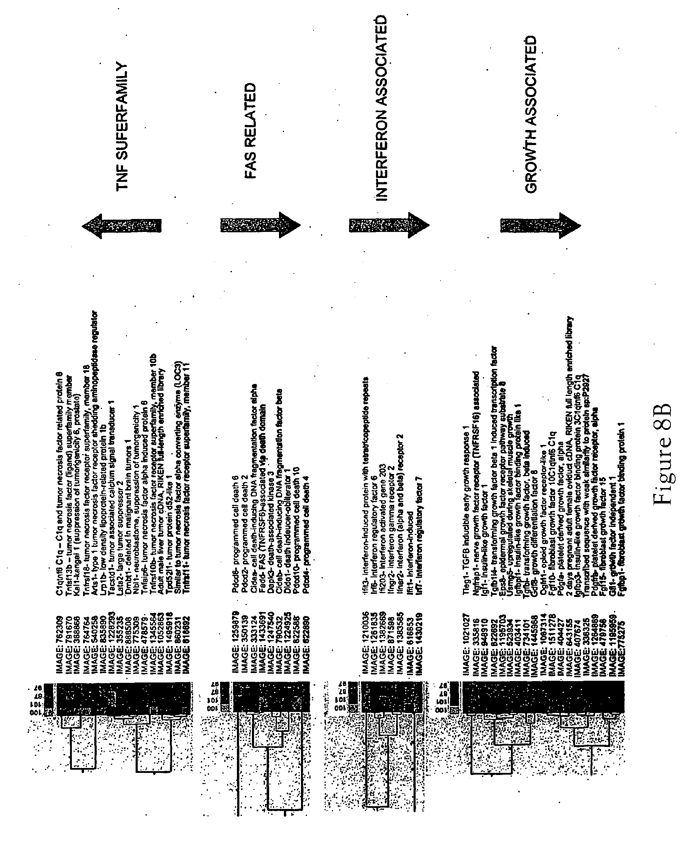

Figures 8A and 8B show gene profiling analysis of SW1 tumors expressing the ATF2⁵¹⁻¹⁰⁰ peptide. Tumors generated, in the absence or presence of a peptide, were used as source of mRNA for array analysis of 10K mouse genes (*see* Example 5). Panel A depicts the overall distribution of the genes found to exhibit altered expression, whereas Panel B provides a list of genes that were found to be induced or repressed upon expression of the ATF2 peptide. In all cases, the data represent analyses carried out 4 times from 4 different pools of tumors.

### DETAILED DESCRIPTION

The present invention provides an approach for inhibiting growth of tumor cells, and particularly for rendering resistant tumor cells susceptible to radiation therapy or chemotherapy. In particular, compositions and methods are provided for altering ATF2 activity to inhibit tumor cell growth, to inhibit tumorgenicity, to sensitize a tumor cell to apoptosis, and to enhance the anti-tumor activity of radiation therapy or chemotherapy. More specifically, amino-terminal fragments of the ATF2 transcription factor are provided that alter ATF2 activity and sensitize tumor cells to spontaneous and induced apoptosis. The present invention is based, in part, on the surprising and unexpected result that ATF2-derived peptides shorter than 50 amino acids (indeed, peptides as short as 10 amino acids in length) were stable and active enough for use in altering the sensitivity of tumor cells (such as human melanoma or breast cancer cells) to radiation and chemical treatment. Moreover, these findings indicate that an ATF2 peptide as short as 10 amino acid (aa) peptide can affect the Jun/JNK signaling cascade *via* altered TRE-dependent activities. Thus, the invention advantageously provides a method for treating a mammal afflicted with a hyperproliferative disease (such as melanoma or breast cancer), in which the cells of the hyperproliferative disease are resistant to spontaneous or induced apoptosis, radiation, chemotherapeutic agents, or any combination thereof.

By way of background and not wishing to be bound by theory, ATF2 along with its interactions with other regulatory components (for example, kinase p38, JNK and c-Jun) is important in a melanoma's resistance to radiation therapy and chemotherapy. ATF2 upregulates the expression of TNF, which serves as a survival factor in late-stage melanoma cells, and p38 attenuates Fas expression via inhibition of NF-κB. The present invention is based, in part, on the surprising result that ATF2-derived peptides can be used to alter the sensitivity of tumor cells (such as human melanoma or breast cancer cells) to radiation and chemical treatment. Initially, four 50 amino acid peptides from the amino-terminal of ATF2 were tested, and the peptide spanning amino acid residues 50-100 elicited the most efficient increase in the sensitivity of human melanoma cells to UV radiation or treatment by mitomycin C, adriamycin and verapamil, or UCN-01, as revealed by apoptosis assays (*see*, *e*.*g*., U.S. Patent Application 2002/0169121). Sensitization by ATF2 peptide was also observed in the MCF7 human breast cancer cells, but not in early-stage melanoma, or melanocytes, or in *in vitro* transformed 293T cells. When combined with an inhibitor of the p38 catalytic activity, cells expressing the 50-100 fragment of ATF2 exhibited an increase in the degree of programmed cell death (both spontaneous and induced apoptosis), indicating that combined targeting of ATF2 and p38 kinases is sufficient to induce apoptosis in, for example, late-stage melanoma cells. The peptide's ability to increase levels of apoptosis coincided with increased cell surface expression of Fas, which is the primary death-signaling cascade in these late stage melanoma cells. Overall, the amino-terminal domain of ATF2 can be used to sensitize tumor cells to radiation and chemical treatment-induced apoptosis, and can be used to induce apoptosis when combined with other chemotherapeutic drugs, such as inhibitors of ATF2 kinase p38.

Any concentration, sequence, quantity, ratio or other numerical range recited herein is to be understood to include any integer within that range and fractions thereof, such as one tenth and one hundredth of an integer, unless otherwise indicated. It should be understood that indefinite terms, such as "a" and "an" as used above and elsewhere herein, refer to "one or more" of the enumerated components, and that the use of the alternative, such as "or," refers to each element individually, collectively or any combination thereof. As used herein, the term "about" means ± 15% of an indicated value.

The invention provides various strategies for altering ATF2 activity, including use of peptides that alter ATF2 activity, use of nucleic acid sequences that encode peptides that alter ATF2 activity, and use of ATF2 RNA interference (RNAi) or antisense olignucleotides. In certain aspects, any of these approaches can be used therapeutically alone, in any combination thereof, or in combination with other therapeutics (*e*.*g*., inducers of apoptosis). The peptide-based approach involves delivering an amino-terminal peptide fragment of ATF2 or an anti-ATF2 antibody to cells, each of which can alter ATF2 activity. In certain embodiments, the amino-terminal peptide fragment of ATF2 or the anti-ATF2 antibody is capable of entering a cell. In still another embodiment, compounds that alter ATF2 activity are combined with a translocation peptide sequence. The vector based approach involves delivering a vector comprising an gene encoding a compound that alters ATF2 activity, such as an amino-terminal peptide fragment of ATF2, an anti-ATF2 antibody, or an ATF2 RNAi or anti-sense nucleic acid sequence.

As used herein, the phrase "compound that alters ATF2 activity" refers to any amino-terminal polypeptide fragment of ATF2 that alters (*i*.*e*., inhibits or enhances, preferably inhibits) ATF2 activity, which excludes full-length ATF2, and is capable of associating with JNK and ranges in size from 2 amino acids up to about 200 amino acids of ATF2. The compound that alters ATF2 activity comprises an amino acid sequence from amino acid residue 51 to amino acid residue 60 (*i*.*e*., a 10 amino acid peptide). The compound that alters ATF2 activity may comprise a JNK association domain with an amino acid sequence from about amino acid residue 51 to about amino acid residue 52; or from about amino acid residue 51 to about amino acid residue 53; or from about amino acid residue 51 to about amino acid residue 54; or from about amino acid residue 51 to about amino acid residue 55. The peptides that alter ATF2 activity may be introduced externally to tumor cells for translocation into the cells, or may be expressed in tumor cells by transfecting expression vectors containing nucleic acid molecules encoding peptides of the instant disclosure.

"Alteration of ATF2 activity" (and all grammatical variations thereof) includes inhibition or enhancement of ATF2-, TRE- or Jun2-dependent transcription. In certain embodiments, the alteration of ATF2 activity comprises inhibition of ATF2-, TRE- or Jun2-dependent transcription or activity, inhibition of tumor cell growth (relative to untreated tumor cells), an enhancement of spontaneous or inducible apoptosis, an increase in the sensitivity of tumor cells to therapy (particularly human melanoma and breast cancer cells), such as UV radiation or treatment by chemotherapeutic drugs (including mitomycin C, adriamycin and verapamil, or UCN-01), and the like. In some embodiments, the alteration of ATF2 activity comprises no longer altering ATF2 or Jun2-dependent transcription and altering TRE-dependent transcription, preferably enhancing TRE-dependent transcription. In certain embodiments, inhibition of ATF2 activity comprises inhibiting growth of a tumor cell, which method comprises inhibiting transcriptional activity of ATF2. In still another embodiment, inhibition of ATF2 activity comprises sensitizing a tumor cell to apoptosis, which method comprises inhibiting the transcriptional activity of ATF2. As used herein, "senstization to apoptosis" or "sensitizing a tumor cell to apoptosis" refers to increasing a cell's susceptibility to entering a programmed cell death (apoptoisis) pathway, including spontaneous (basal) or induced apoptoisis. In certain embodiments, the tumor cells being sensitized to apoptosis are resistant to apoptosis, such as late stage melanoma cells or breast cancer cells.

As used herein, the term "tumor" refers to a malignant tissue comprising transformed cells that grow uncontrollably (*i*.*e*., is a hyperproliferative disease). Tumors include leukemias, lymphomas, myelomas, plasmacytomas, and the like; and solid tumors. Examples of solid tumors that can be treated according to the invention include sarcomas and carcinomas such as: melanoma, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, neuroblastoma, and retinoblastoma.

As used herein, the term "mammal" has its ordinary meaning, and specifically includes primates, and more specifically includes humans. Other mammals that may be treated for the presence of a tumor, or in which tumor cell growth may be inhibited, include canine, feline, rodent (racine, murine, lupine, *etc*.), equine, bovine, ovine, caprine, or porcine species.

"Gene therapy" refers to transfer of a gene encoding an effector molecule into cells, in this case of the tumor. Gene therapy vectors include, but are not limited to, viral vectors (including retroviruses and DNA viruses), naked DNA vectors, and DNA-transfection agent admixtures. Such methods, including routes of administration and dose, are well known in the art.

### Translocation Peptide Sequences

Peptide sequences that mediate membrane transport and, accordingly, provide for delivery of polypeptides to the cytoplasm are known in the art. For example, such peptides can be derived from the antennapedia homeodomain helix 3 to generate membrane transport vectors, such as penetratin (PCT Publication WO 00/29427; *see also* Fischer et al., J. Pept. Res. 2000, 55:163-72; DeRossi et al., Trends in Cell Biol. 1998, 8:84-7; Brugidou et al., Biochem. Biophys. Res. Comm. 1995, 214:685-93). Protein translocation domains, which include the antennapedia domain and the HIV-TAT domain (*see* Vives et al., J. Biol. Chem. 1997, 272:16010-17), generally posses a characteristic positive charge, which led to the development of cationic 12-mer peptides that are useful for transfering therapeutic compounds, such as peptides, polypeptides or nucleic acids, into cells (Mi et al., Mol. Therapy 2000, 2:339-47). As used herein, a "translocation peptide sequence" comprises an amino acid sequence capable of faciliting the passage of compounds associated with the translocation sequence across cell membranes. For example, a translocation peptide sequence can aid an ATF2 peptide of this disclosure to pass across a plasma membrane from outside a cell to inside a cell, or pass across the nuclear membrane from the cell cytoplasm into the nucleus, or pass across a mitochondrial memebrane from the cell cytoplasm into a mitochondrion. In certain embodiments, the translocation peptide sequence comprises HA-penetratin (*see, e*.*g*., Bhoumik et al., Clin. Cancer Res., 2001;2:331-342) or HIV-TAT (*see, e*.*g*., Vocero-Akbani et al., Methods Enzymol., 2000, 322:508-21), and the compound that alters ATF2 activity is any of the amino-terminal fragments of ATF2 as described herein i.e. ATF2⁵¹⁻⁶⁰.

Therapeutic peptides or polypeptides can be generated by creating fusion proteins or polypeptide conjugates combining a translocation peptide sequence with a therapeutically functional sequence. For example, p21^{WAF1}-derived peptides linked to a translocation peptide inhibited ovarian tumor cell line growth (Bonfanti et al., Cancer Res. 1997, 57:1442-1446). These constructs yield more stable drug-like polypeptides able to penetrate cells and able to effect a therapeutic outcome. These constructs can also form the basis for rational drug design approaches.

In a certain embodiments, a compound that alters ATF2 activity is combined with a peptide translocation sequence, preferably the compound and translocation sequence are recombinantly fused to form a fusion protein. The fusion protein can be prepared synthetically or recombinantly. In particular embodinments, the compound that alters ATF2 activity is any of the amino-terminal fragments of ATF2 as described herein.

An alternative approach employs an anti-ATF2 antibody combined, fused or conjugated to a peptide translocation sequence, which can be administered systemically or locally for intracellular activity. Preferably, such an anti-ATF2 antibody is a single chain Fv antibody.

### ATF2 Peptide Antibodies

Intracellular antibodies (sometime referred to as "intrabodies") have been used to regulate the activity of intracellular proteins in a number of systems (see, Marasco, Gene Ther. 1997, 4:11; Chen et al., Hum. Gene Ther. 1994, 5:595), *e*.*g*., viral infections (Marasco et al., Hum. Gene Ther., 1998, 9:1627) and other infectious diseases (Rondon et al., Annu. Rev. Microbiol., 1997, 51:257), and oncogenes, such as p21 (Cardinale et al., FEBS Lett. 1998, 439:197-202; and Cochet et al., Cancer Res., 1998, 58:1170-6), myb (Kasono et al., Biochem Biophys Res Commun., 1998, 251:124-30), erbB-2 (Graus-Porta et al., Mol Cell Biol., 1995, 15:1182-91), etc. This technology can be adapted to alter ATF2 activity by expression of an anti-ATF2 intracellular antibody.

Alternatively, monoclonal antibodies directed toward the ATF2 polypeptide, or fragment, analog, or derivative thereof, may be used, provided they are directed into the cytoplasm of the cell to bind and alter ATF2 activity. Methods of obtaining such antibodies include the hybridoma technique originally developed by Kohler and Milstein (Nature 1975, 256:495-497), as well as the trioma technique, the human B-cell hybridoma technique (Kozbor et al., Immunology Today 1983, 4:72; Cote et al., Proc. Nat'l. Acad. Sci. USA, 1983, 80:2026-2030), and the EBV-hybridoma technique to produce human monoclonal antibodies (Cole et al., in Monoclonal Antibodies and Cancer Therapy, Alan R. Liss, Inc., pp. 77-96, 1985). Monoclonal antibodies can be produced in germ-free animals (PCT Publication No. WO 89/12690). In fact, according to the invention, techniques developed for the production of "chimeric antibodies" (Morrison et al., J. Bacteriol., 1984, 159:870; Neuberger et al., Nature, 1984, 312:604-608; and Takeda et al., Nature, 1985, 314:452-454) by splicing the genes from a mouse antibody molecule specific for an ATF2 polypeptide together with genes from a human antibody molecule of appropriate biological activity can be used; such antibodies are disclosed herein . Such human or humanized chimeric antibodies are preferred for use in therapy of human diseases or disorders (as described herein) because the human or humanized antibodies are much less likely than xenogenic antibodies to induce an immune response, in particular an allergic response, themselves.

Techniques described for the production of single chain antibodies (U.S. Patent Nos. 5,476,786 and 5,132,405 to Huston; U.S. Patent 4,946,778) can be adapted to produce ATF2 polypeptide-specific single chain antibodies. Indeed, these genes can be delivered for expression *in vivo.* The techniques described for the construction of Fab expression libraries (Huse et al., Science 246:1275-1281, 1989) allow rapid and easy identification of monoclonal Fab fragments with the desired specificity for an ATF2 polypeptide, or its derivatives, or analogs. Single chain antibodies (which for the basis for most intrabody technology) are preferred, particularly those engineered to express a peptide translocation sequence.

Antibody fragments which contain the idiotype of the antibody molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, and the Fab fragments which can be generated by treating the antibody molecule with papain and a reducing agent.

### ATF2 Peptide-Encoding Nucleic Acids

In addition to ATF2 peptide expressing vectors, which are described in detail herein, the present disclosure contemplates that RNAi and antisense nucleic acids, such as DNA, RNA, nucleic acid analogs (as described herein) and the like, can be used to alter ATF2 activity, such as by inhibiting expression of ATF2.

### Molecular Biology - Definitions

In accordance with the present invention there may be employed conventional molecular biology, microbiology, and recombinant DNA techniques within the skill of the art. Such techniques are explained fully in the literature. See, *e*.*g*., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription And Translation [B.D. Hames & S.J. Higgins, eds. (1984)]; Animal Cell Culture [R.I. Freshney, ed. (1986)]; Immobilized Cells And Enzymes [IRL Press, (1986)]; B. Perbal, A Practical Guide To Molecular Cloning (1984); F.M. Ausubel et al. (eds.), Current Protocols in Molecular Biology, John Wiley & Sons, Inc. (1994).

A "polynucleotide" or "nucleotide sequence" is a series of nucleotide bases (also called "nucleotides") in a nucleic acid, such as DNA and RNA, and means any chain of two or more nucleotides. A nucleotide sequence typically carries genetic information, including the information used by cellular machinery to make proteins and enzymes. These terms include double or single stranded genomic and cDNA, RNA, any synthetic and genetically manipulated polynucleotide, and both sense and anti-sense polynucleotide (although only sense stands are being represented herein). This includes single- and double-stranded molecules, *i*.*e*., DNA-DNA, DNA-RNA and RNA-RNA hybrids, as well as "protein nucleic acids" (PNA) formed by conjugating bases to an amino acid backbone. This also includes nucleic acids containing modified bases, for example thio-uracil, thio-guanine and fluoro-uracil.

The nucleic acids herein may be flanked by natural regulatory (expression control) sequences, or may be associated with heterologous sequences, including promoters, internal ribosome entry sites (IRES) and other ribosome binding site sequences, enhancers, response elements, suppressors, signal sequences, polyadenylation sequences, introns, 5'- and 3'- non-coding regions, and the like. The nucleic acids may also be modified by many means known in the art. Non-limiting examples of such modifications include methylation, "caps", substitution of one or more of the naturally occurring nucleotides with an analog, and internucleotide modifications such as, for example, those with uncharged linkages (*e*.*g*., methyl phosphonates, phosphotriesters, phosphoroamidates, carbamates, etc.) and with charged linkages (*e*.*g*., phosphorothioates, phosphorodithioates, etc.). Polynucleotides may contain one or more additional covalently linked moieties, such as, for example, proteins (*e*.*g*., nucleases, toxins, antibodies, signal peptides, poly-L-lysine, etc.), intercalators (*e*.*g*., acridine, psoralen, etc.), chelators (*e*.*g*., metals, radioactive metals, iron, oxidative metals, etc.), and alkylators. The polynucleotides may be derivatized by formation of a methyl or ethyl phosphotriester or an alkyl phosphoramidate linkage. Furthermore, the polynucleotides herein may also be modified with a label capable of providing a detectable signal, either directly or indirectly. Exemplary labels include radioisotopes, fluorescent molecules, biotin, and the like.

A "promoter" or "promoter sequence" is a DNA regulatory region capable of binding RNA polymerase in a cell and initiating transcription of a downstream (3' direction) coding sequence. For purposes of defining the present invention, a promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined for example, by mapping with nuclease S1), as well as protein binding domains (consensus sequences) responsible for the binding of RNA polymerase. The promoter may be operably associated with other expression control sequences, including enhancer and repressor sequences.

Promoters which may be used to control gene expression include, but are not limited to, elongation factor promoter from polyoma virus, cytomegalovirus (CMV) promoter (U.S. Patents No. 5,385,839 and No. 5,168,062), the SV40 early promoter region (Benoist and Chambon, Nature 1981, 290:304-310), the promoter contained in the 3' long terminal repeat of Rous sarcoma virus (Yamamoto, et al., Cell 1980, 22:787-797), the herpes thymidine kinase promoter (Wagner et al., Proc. Natl. Acad. Sci. USA 1981, 78:1441-1445), the regulatory sequences of the metallothionein gene (Brinster et al., Nature 1982, 296:39-42); prokaryotic expression vectors such as the beta-lactamase promoter (Villa-Komaroff, et al., Proc. Natl. Acad. Sci. USA 1978, 75:3727-3731), or the *tac* promoter (DeBoer, et al., Proc. Natl. Acad. Sci. USA 1983, 80:21-25); see also "Useful proteins from recombinant bacteria" in Scientific American 1980, 242:74-94; promoter elements from yeast or other fungi such as the Gal 4 promoter, the ADC (alcohol dehydrogenase) promoter, PGK (phosphoglycerol kinase) promoter, alkaline phosphatase promoter; and transcriptional control regions that exhibit hematopoietic tissue specificity, in particular: beta-globin gene control region which is active in myeloid cells (Mogram et al., Nature 1985, 315:338-340; Kollias et al., Cell 1986, 46:89-94), hematopoietic stem cell differentiation factor promoters, erythropoietin receptor promoter (Maouche et al., Blood 1991,15:2557), etc. Inducible/repressible promoter systems can also be used, such as the tet, RU 486, and echdysone inducible systems, and the tet repressor system.

A "coding sequence" or a sequence "encoding" an expression product, such as a RNA, polypeptide, protein, or enzyme, is a nucleotide sequence that, when expressed, results in the production of that RNA, polypeptide, protein, or enzyme, *i*.*e*., the nucleotide sequence encodes an amino acid sequence for that polypeptide, protein or enzyme. A coding sequence for a protein may include a start codon (usually ATG) and a stop codon.

The term "gene", also called a "structural gene" means a DNA sequence that codes for or corresponds to a particular sequence of amino acids which comprise all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription.

A coding sequence is "under the control of" or "operably or operatively associated with" transcriptional and translational control sequences in a cell when RNA polymerase transcribes the coding sequence into RNA, particularly mRNA, which is then trans-RNA spliced (if it contains introns) and translated into the protein encoded by the coding sequence.

The terms "vector", "cloning vector" and "expression vector" mean the vehicle by which a DNA or RNA sequence (*e*.*g*. a foreign gene) can be introduced into a host cell, so as to transform the host and promote expression (*e*.*g*. transcription and translation) of the introduced sequence. Vectors include plasmids, phages, viruses, etc.; they are discussed in greater detail below.

Vectors typically comprise the DNA of a transmissible agent, into which foreign DNA is inserted. A common way to insert one segment of DNA into another segment of DNA involves the use of enzymes called restriction enzymes that cleave DNA at specific sites (specific groups of nucleotides) called restriction sites. A "cassette" refers to a DNA coding sequence or segment of DNA that codes for an expression product that can be inserted into a vector at defined restriction sites. The cassette restriction sites are designed to ensure insertion of the cassette in the proper reading frame. Generally, foreign DNA is inserted at one or more restriction sites of the vector DNA, and then is carried by the vector into a host cell along with the transmissible vector DNA. A segment or sequence of DNA having inserted or added DNA, such as an expression vector, can also be called a "DNA construct." A common type of vector is a "plasmid", which generally is a self-contained molecule of double-stranded DNA, usually of bacterial origin, that can readily accept additional (foreign) DNA and which can readily introduced into a suitable host cell. A plasmid vector often contains coding DNA and promoter DNA and has one or more restriction sites suitable for inserting foreign DNA. Coding DNA is a DNA sequence that encodes a particular amino acid sequence for a particular protein or enzyme. Promoter DNA is a DNA sequence which initiates, regulates, or otherwise mediates or controls the expression of the coding DNA. Promoter DNA and coding DNA may be from the same gene or from different genes, and may be from the same or different organisms. A large number of vectors, including plasmids and fungal vectors, have been described for replication and/or expression in a variety of eukaryotic and prokaryotic hosts. Non-limiting examples include pKK plasmids (Clonetech), pUC plasmids, pET plasmids (Novagen, Inc., Madison, WI), pRSET or pREP plasmids (Invitrogen, San Diego, CA), pQE plasmids (Qiagen, Chatsworth, CA), or pMAL plasmids (New England Biolabs, Beverly, MA), and many appropriate host cells, using methods disclosed or cited herein or otherwise known to those skilled in the relevant art. Recombinant cloning vectors will often include one or more replication systems for cloning or expression, one or more markers for selection in the host, *e*.*g*. antibiotic resistance, one or more tags or fuion sequence (such as a 6 x histidine tag or FLAG epitope),or one or more expression cassettes.

The terms "express" and "expression" mean allowing or causing the information in a gene or DNA sequence to become manifest, for example producing a protein by activating the cellular functions involved in transcription and translation of a corresponding gene or DNA sequence. A DNA sequence is expressed in or by a cell to form an "expression product" such as a protein. The expression product itself, *e*.*g*. the resulting protein, may also be said to be "expressed" by the cell. An expression product can be characterized as intracellular, extracellular or secreted. The term "intracellular" means something that is inside a cell. The term "extracellular" means something that is outside a cell. A substance is "secreted" by a cell if it appears in significant measure outside the cell, from somewhere on or inside the cell.

The term "transfection" means the introduction of a foreign nucleic acid into a cell. The term "transformation" means the introduction of a "foreign" (*i*.*e*. extrinsic or extracellular) gene, DNA or RNA sequence to a host cell, so that the host cell will express the introduced gene or sequence to produce a desired substance, topically a protein or enzyme coded by the introduced gene or sequence. The introduced gene or sequence may also be called a "cloned" or "foreign" gene or sequence, may include regulatory or control sequences, such as start, stop, promoter, signal, secretion, or other sequences used by a cell's genetic machinery. The gene or sequence may include nonfunctional sequences or sequences with no known function. A host cell that receives and expresses introduced DNA or RNA has been "transformed" and is a "transformant" or a "clone." The DNA or RNA introduced to a host cell can come from any source, including cells of the same genus or species as the host cell, or cells of a different genus or species.

The term "host cell" means any cell of any organism that is selected, modified, transformed, grown, or used or manipulated in any way, for the production of a substance by the cell, for example the expression by the cell of a gene, a DNA or RNA sequence, a protein or an enzyme. Host cells can further be used for screening or other assays, as described *infra*.

The term "expression system" means a host cell and compatible vector under suitable conditions, *e*.*g*. for the expression of a protein coded for by foreign DNA carried by the vector and introduced to the host cell. Common expression systems include *E. coli* host cells and plasmid vectors, insect host cells and *Baculovirus* vectors, and mammalian host cells and vectors. In a specific embodiment, the protein of interest is expressed in COS-1 or C₂C₁₂ cells. Other suitable cells include CHO cells, HeLa cells, 293T (human kidney cells), mouse primary myoblasts, and NIH 3T3 cells.

The term "heterologous" refers to a combination of elements not naturally occurring. For example, heterologous DNA refers to DNA not naturally located in the cell, or in a chromosomal site of the cell. Preferably, the heterologous DNA includes a gene foreign to the cell. A heterologous expression regulatory element is such an element operatively associated with a different gene than the one it is operatively associated with in nature. In the context of the present invention, a gene encoding a protein of interest is heterologous to the vector DNA in which it is inserted for cloning or expression, and it is heterologous to a host cell containing such a vector, in which it is expressed, *e*.*g*., a CHO cell.

The terms "mutant" and "mutation" mean any detectable change in genetic material, *e*.*g*., DNA, or any process, mechanism, or result of such a change. This includes gene mutations, in which the structure (*e*.*g*., DNA sequence) of a gene is altered, any gene or DNA arising from any mutation process, and any expression product (*e*.*g*., protein or enzyme) expressed by a modified gene or DNA sequence. The term "variant" may also be used to indicate a modified or altered gene, DNA sequence, enzyme, cell, etc., *i*.*e*., any kind of mutant.

"Sequence-conservative variants" of a polynucleotide sequence are those in which a change of one or more nucleotides in a given codon position results in no alteration in the amino acid encoded at that position.

"Function-conservative variants" are those in which a given amino acid residue in a protein or enzyme has been changed without altering the overall conformation and function of the polypeptide, including replacement of an amino acid with one having similar properties (such as, for example, polarity, hydrogen bonding potential, acidic, basic, hydrophobic, aromatic, and the like). Amino acids with similar properties are well known in the art. For example, arginine, histidine and lysine are hydrophilic-basic amino acids and may be interchangeable. Similarly, isoleucine, a hydrophobic amino acid, may be replaced with leucine, methionine or valine. Such changes are expected to have little or no effect on the apparent molecular weight or isoelectric point of the protein or polypeptide. Amino acids other than those indicated as conserved may differ in a protein or enzyme so that the percent protein or amino acid sequence similarity between any two proteins of similar function may vary and may be, for example, from 70% to 99% as determined according to an alignment scheme such as by the Cluster Method, wherein similarity is based on the MEGALIGN algorithm. A "function- conservative variant" also includes a polypeptide or enzyme which has at least 60 % amino acid identity as determined by BLAST or FASTA algorithms, preferably at least 75%, most preferably at least 85%, and even more preferably at least 90%, and which has the same or substantially similar properties or functions as the native or parent protein or enzyme to which it is compared.

As used herein, the term "homologous" in all its grammatical forms and spelling variations refers to the relationship between proteins that possess a "common evolutionary origin," including proteins from superfamilies (*e*.*g*., the immunoglobulin superfamily) and homologous proteins from different species (*e*.*g*., myosin light chain, etc.) (Reeck et al., Cell 50:667, 1987). Such proteins (and their encoding genes) have sequence homology, as reflected by their sequence similarity, whether in terms of percent similarity or the presence of specific residues or motifs at conserved positions.

Accordingly, the term "sequence similarity" in all its grammatical forms refers to the degree of identity or correspondence between nucleic acid or amino acid sequences of proteins that may or may not share a common evolutionary origin (*see* Reeck et al., Cell 50:667, 1987). However, in common usage and in the instant application, the term "homologous," when modified with an adverb such as "highly," may refer to sequence similarity and may or may not relate to a common evolutionary origin.

In a specific embodiment, two DNA sequences are "substantially homologous" or "substantially similar" when at least about 80%, and most preferably at least about 90 or 95%) of the nucleotides match over the defined length of the DNA sequences, as determined by sequence comparison algorithms, such as BLAST, FASTA, DNA Strider, etc. An example of such a sequence is an allelic or species variant of the specific genes of the invention. Sequences that are substantially homologous can be identified by comparing the sequences using standard software available in sequence data banks, or in a Southern hybridization experiment under, for example, stringent conditions as defined for that particular system.

Similarly, in a particular embodiment, two amino acid sequences are "substantially homologous" or "substantially similar" when greater than 80% of the amino acids are identical, or greater than about 90% are similar (functionally identical). Preferably, the similar or homologous sequences are identified by alignment using, for example, the GCG (Genetics Computer Group, Program Manual for the GCG Package, *Version* 7, Madison, Wisconsin) pileup program, or any of the programs described above (BLAST, FASTA, etc.).

A nucleic acid molecule is "hybridizable" to another nucleic acid molecule, such as a cDNA, genomic DNA, or RNA, when a single stranded form of the nucleic acid molecule can anneal to the other nucleic acid molecule under the appropriate conditions of temperature and solution ionic strength (*see* Sambrook *et al*., *supra*). The conditions of temperature and ionic strength determine the "stringency" of the hybridization. For preliminary screening for homologous nucleic acids, low stringency hybridization conditions, corresponding to a Tₘ (melting temperature) of 55°C, can be used, *e*.*g*., 5x SSC, 0.1% SDS, 0.25% milk, and no formamide; or 30% formamide, 5x SSC, 0.5% SDS). Moderate stringency hybridization conditions correspond to a higher Tₘ, *e*.*g*., 40% formamide, with 5x or 6x SCC. High stringency hybridization conditions correspond to the highest Tₘ, *e.g.,* 50% formamide, 5x or 6x SCC. SCC is a 0.15 M NaCl, 0.015 M Na-citrate. Hybridization requires that the two nucleic acids contain complementary sequences, although depending on the stringency of the hybridization, mismatches between bases are possible. The appropriate stringency for hybridizing nucleic acids depends on the length of the nucleic acids and the degree of complementation, variables well known in the art. The greater the degree of similarity or homology between two nucleotide sequences, the greater the value of Tₘ for hybrids of nucleic acids having those sequences. The relative stability (corresponding to higher Tₘ) of nucleic acid hybridizations decreases in the following order: RNA:RNA, DNA:RNA, DNA:DNA. For hybrids of greater than 100 nucleotides in length, equations for calculating Tₘ have been derived (*see* Sambrook *et al*., *supra*, 9.50-9.51). For hybridization with shorter nucleic acids, *i*.*e*., oligonucleotides, the position of mismatches becomes more important, and the length of the oligonucleotide determines its specificity *(see* Sambrook *et al*., *supra*, 11.7-11.8). A minimum length for a hybridizable nucleic acid is at least about 10 nucleotides; preferably at least about 15 nucleotides; and more preferably the length is at least about 20 nucleotides.

In a specific embodiment, the term "standard hybridization conditions" refers to a Tₘ of 55°C, and utilizes conditions as set forth above. In a preferred embodiment, the Tₘ is 60°C; in a more preferred embodiment, the Tₘ is 65°C. In a specific embodiment, "high stringency" refers to hybridization and/or washing conditions at 68°C in 0.2XSSC, at 42°C in 50% formamide, 4XSSC, or under conditions that afford levels of hybridization equivalent to those observed under either of these two conditions.

As used herein, the term "oligonucleotide" refers to a nucleic acid, generally of at least 10, preferably at least 15, and more preferably at least 20 nucleotides, preferably no more than 100 nucleotides, that is hybridizable to a genomic DNA molecule, a cDNA molecule, or an mRNA molecule encoding a gene, mRNA, cDNA, or other nucleic acid of interest. Oligonucleotides can be labeled, *e*.*g*., with ³²P-nucleotides or nucleotides to which a label, such as biotin, has been covalently conjugated. In one embodiment, a labeled oligonucleotide can be used as a probe to detect the presence of a nucleic acid. In another embodiment, oligonucleotides (one or both of which may be labeled) can be used as PCR primers, either for cloning full length or a fragment of the gene, or to detect the presence of nucleic acids encoding the protein. In a further embodiment, an oligonucleotide of the invention can form a triple helix with a DNA molecule. Generally, oligonucleotides are prepared synthetically, preferably on a nucleic acid synthesizer. Accordingly, oligonucleotides can be prepared with non-naturally occurring phosphoester analog bonds, such as thioester bonds, etc.

The present invention provides antisense nucleic acids (including ribozymes), which may be used to inhibit expression of a target protein of the invention. An "antisense nucleic acid" is a single stranded nucleic acid molecule which, on hybridizing under cytoplasmic conditions with complementary bases in an RNA or DNA molecule, inhibits the latter's role. If the RNA is a messenger RNA transcript, the antisense nucleic acid is a countertranscript or mRNA-interfering complementary nucleic acid. As presently used, "antisense" broadly includes RNA-RNA interactions, RNA-DNA interactions, ribozymes and RNase-H mediated arrest. Antisense nucleic acid molecules can be encoded by a recombinant gene for expression in a cell (*e*.*g*., U.S. Patent No. 5,814,500; U.S. Patent No. 5,811,234), or alternatively they can be prepared synthetically (*e*.*g*., U.S. Patent No. 5,780,607).

Specific non-limiting examples of synthetic oligonucleotides envisioned for this invention include oligonucleotides thatcontain phosphorothioates, phosphotriesters, methyl phosphonates, short chain alkyl, or cycloalkl intersugar linkages or short chain heteroatomic or heterocyclic intersugar linkages. Most preferred are those with CH₂-NH-O-CH₂, CH₂-N(CH₃)-O-CH₂, CH₂-O-N(CH₃)-CH₂, CH₂-N(CH₃)-N(CH₃)-CH₂ and O-N(CH₃)-CH₂-CH₂ backbones (where phosphodiester is O-PO₂-O-CH₂). US Patent No. 5,677,437 describes heteroaromatic olignucleoside linkages. Nitrogen linkers or groups containing nitrogen can also be used to prepare oligonucleotide mimics (U.S. Patents No. 5,792,844 and No. 5,783,682). U.S. Patent No. 5,637,684 describes phosphoramidate and phosphorothioamidate oligomeric compounds. Also envisioned are oligonucleotides having morpholino backbone structures (U.S. Patent No. 5,034,506). In other embodiments, such as the peptide-nucleic acid (PNA) backbone, the phosphodiester backbone of the oligonucleotide may be replaced with a polyamide backbone, the bases being bound directly or indirectly to the aza nitrogen atoms of the polyamide backbone (Nielsen et al., Science 1991, 254:1497). Other synthetic oligonucleotides may contain substituted sugar moieties comprising one of the following at the 2' position: OH, SH, SCH₃, F, OCN, O(CH₂)ₙNH₂ or 0(CH₂)ₙCH₃ where n is from 1 to about 10; C₁ to C₁₀ lower alkyl, substituted lower alkyl, alkaryl or aralkyl; Cl; Br; CN; CF₃; OCF₃; O-; S-, or N-alkyl; O-, S-, or N-alkenyl; SOCH₃ ; SO₂CH₃; ONO₂;NO₂; N₃; NH₂; heterocycloalkyl; heterocycloalkaryl; aminoalkylamino; polyalkylamino; substitued silyl; a fluorescein moiety; an RNA cleaving group; a reporter group; an intercalator; a group for improving the pharmacokinetic properties of an oligonucleotide; or a group for improving the pharmacodynamic properties of an oligonucleotide, and other substituents having similar properties. Oligonucleotides may also have sugar mimetics such as cyclobutyls or other carbocyclics in place of the pentofuranosyl group. Nucleotide units having nucleosides other than adenosine, cytidine, guanosine, thymidine and uridine, such as inosine, may be used in an oligonucleotide molecule.

### Expression Vectors

Preferred vectors *in vitro*, *in vivo,* and *ex vivo* are viral vectors, such as lentiviruses, retroviruses, herpes viruses, adenoviruses, adeno-associated viruses, vaccinia virus, baculovirus, and other recombinant viruses with desirable cellular tropism. Thus, a gene encoding a functional or mutant protein or polypeptide domain fragment thereof can be introduced *in vivo, ex vivo,* or *in vitro* using a viral vector or through direct introduction of DNA. Expression in targeted tissues can be effected by targeting the transgenic vector to specific cells, such as with a viral vector or a receptor ligand, or by using a tissue-specific promoter, or both. Targeted gene delivery is described in PCT Publication WO 95/28494.

Viral vectors commonly used for *in vivo* or *ex vivo* targeting and therapy procedures are DNA-based vectors and retroviral vectors. Methods for constructing and using viral vectors are known in the art (*see, e.g*., Miller and Rosman, BioTechniques 1992, 7:980-990). Preferably, the viral vectors are replication defective, that is, they are unable to replicate autonomously in the target cell. Preferably, the replication defective virus is a minimal virus, *i*.*e*., it retains only the sequences of its genome which are necessary for encapsidating the genome to produce viral particles.

The gene can be introduced in a retroviral vector, *e*.*g*., as described in Anderson et al., U.S. Patent No. 5,399,346; Mann et al., Cell 1983, 33:153; U.S. Patent Nos. 4,650,764, 4,980,289, and 5,124,263; Markowitz et al., J. Virol.1988, 62:1120; Temin *et al*., U.S. Patent No.; EP 453242, EP178220; Bernstein et al. Genet. Eng. 1985, 7:235; McCormick, BioTechnology 1985, 3:689; PCT; and Kuo et al., 1993, Blood 82:845. These vectors can be constructed from different types of retrovirus, such as, HIV, MoMuLV ("murine Moloney leukaemia virus" MSV ("murine Moloney sarcoma virus"), HaSV ("Harvey sarcoma virus"); SNV ("spleen necrosis virus"); RSV ("Rous sarcoma virus") and Friend virus. Suitable packaging cell lines have been described in the prior art, in particular the cell line PA317 (US Patent No. 4,861,719); the PsiCRIP cell line (PCT Publication No. WO 90/02806) and the GP+envAm-12 cell line (PCT Publication No. WO 89/07150). Retrovirus vectors can also be introduced by DNA viruses, which permits one cycle of retroviral replication and amplifies tranfection efficiency (*see* PCT Publication Nos. WO 95/22617, WO 95/26411, WO 96/39036, WO 97/19182).

In another embodiment, lentiviral vectors are can be used as agents for the direct delivery and sustained expression of a transgene in several tissue types, including brain, retina, muscle, liver and blood. The vectors can efficiently transduce dividing and nondividing cells in these tissues, and maintain long-term expression of the gene of interest (*see* Naldini, Curr. Opin. Biotechnol., 9:457-63, 1998; *see also* Zufferey, et al., J. Virol., 72:9873-80, 1998; Kafri, et al., J. Virol., 73: 576-584, 1999).

DNA viral vectors include an attenuated or defective DNA virus, such as herpes simplex virus (HSV), papillomavirus, Epstein Barr virus (EBV), adenovirus, adeno-associated virus (AAV), and the like. Defective viruses, which entirely or almost entirely lack viral genes, are preferred. Defective virus is not infective after introduction into a cell. Use of defective viral vectors allows for administration to cells in a specific, localized area, without concern that the vector can infect other cells. Thus, a specific tissue can be specifically targeted. Examples of particular vectors include, but are not limited to, a defective herpes virus 1 (HSV1) vector (Kaplitt et al., Molec. Cell. Neurosci. 1991, 2:320-330), defective herpes virus vector lacking a glyco-protein L gene (Patent. Publication RD 371005 A), or other defective herpes virus vectors (PCT Publication Nos. WO 94/21807 and WO 92/05263); an attenuated adenovirus vector, such as the vector described by Stratford-Perricaudet et al. (J. Clin. Invert., 1992, 90:626-630); *see also* La Salle et al., Science, 1993, 259:988-990; various replication defective adenovirus and minimum adenovirus vectors have been described in PCT Publication Nos. WO 94/26914, WO 95/02697, WO 94/28938, WO 94/28132, WO 94/12649, WO 95/02697, and WO 96/22378); and a defective adeno-associated virus vector (Samulski et al., J. Virol. 1987, 61:3096-3101; Samulski et al., J. Virol. 1989, 63:3822-3828; Lebkowski et al., Mol. Cell. Biol., 1988, 8:3988-3996; PCT Publication Nos. WO 91/18088 and WO 93/09239; US Patent Nos. 4,797,368 and 5,139,941; European Publication No. EP 488 528).

Preferably, for *in vivo* administration, an appropriate immunosuppressive treatment is employed in conjunction with the viral vector, *e.g.*, adenovirus vector, to avoid immuno-deactivation of the viral vector and transfected cells. For example, immunosuppressive cytokines, such as interleukin-12 (IL-12), interferon- (IFN-), or anti-CD4 antibody, can be administered to block humoral or cellular immune responses to the viral vectors (*see, e*.*g*., Wilson, Nature Medicine, 1995). In that regard, it is advantageous to employ a viral vector that is engineered to express a minimal number of antigens.

Various companies produce viral vectors commercially, including but by no means limited to Avigen, Inc. (Alameda, CA; AAV vectors), Cell Genesys (Foster City, CA; retroviral, adenoviral, AAV vectors, and lentiviral vectors), Clontech (retroviral and baculoviral vectors), Genovo, Inc. (Sharon Hill, PA; adenoviral and AAV vectors), Genvec (adenoviral vectors), IntroGene (Leiden, Netherlands; adenoviral vectors), Molecular Medicine (retroviral, adenoviral, AAV, and herpes viral vectors), Norgen (adenoviral vectors), Oxford BioMedica (Oxford, United Kingdom; lentiviral vectors), and Transgene (Strasbourg, France; adenoviral, vaccinia, retroviral, and lentiviral vectors).

In another embodiment, the vector can be non-viral. Such vectors include "naked" DNA, and transfection facilitating agents (peptides, polymers, etc.). Synthetic cationic lipids can be used to prepare liposomes for transfection of a gene encoding (Felgner et. al., Proc. Natl. Acad Sci. USA. 84:7413-7417, 1987; Felgner and Ringold, Science 337:387-388, 1989; see Mackey et al., Proc. Natl. Acad Sci. U.S.A. 85:8027-8031, 1988; Ulmer et al., Science 259:1745-1748, 1993). Useful lipid compounds and compositions for transfer of nucleic acids are described in International Patent Publications WO95/18863 and WO96/17823, and in U.S. Patent No. 5,439,127. Lipids may be chemically coupled to other molecules for the purpose of targeting (see Mackey et. al., Proc. Natl. Acad Sci. U.S.A. 85:8027-8031). Targeted peptides, *e*.*g*., hormones or neurotransmitters, and proteins such as antibodies, or non-peptide molecules could be coupled to liposomes chemically. Other molecules are also useful for facilitating transfection of a nucleic acid *in vivo,* such as a cationic oligopeptide (*e.g.*, International Patent Publication WO95/21931), peptides derived from DNA binding proteins (*e.g.*, International Patent Publication WO96/25508), or a cationic polymer (*e*.*g*., International Patent Publication WO95/21931).

It is also possible to introduce the vector as a naked DNA plasmid. Naked DNA vectors for gene therapy can be introduced into the desired host cells by methods known in the art, *e.g.*, electroporation, microinjection, cell fusion, DEAE dextran, calcium phosphate precipitation, use of a gene gun, or use of a DNA vector transporter (see, e.g., Wu et al., J. Biol. Chem. 267:963-967, 1992; Wu and Wu, J. Biol. Chem. 263:14621-14624, 1988; Hartmut *et al*., Canadian Patent Application No. 2,012,311, filed March 15,1990; and Williams et al., Proc. Nat'l. Acad Sci. USA 88:2726-2730, 1991). Receptor-mediated DNA delivery approaches can also be used (Curiel et al., Hum. Gene Ther. 3:147-154, 1992; and Wu and Wu, J. Biol. Chem. 262:4429-4432, 1987). U.S. Patent Nos. 5,580,859 and 5,589,466 disclose delivery of exogenous DNA sequences, free of transfection facilitating agents, in a mammal. Recently, a relatively low voltage, high efficiency *in vivo* DNA transfer technique, termed electrotransfer, has been described (Mir et al., C.P. Acad Sci., 321:893, 1998; WO 99/01157; WO 99/01158; and WO 99/01175).

### ATF2 Peptide Therapy

As noted above, strategies for altering ATF2 activity can be used to treat any cancer in which tumor cells demonstrate resistance to apoptosis, radiation, chemotherapeutic agents, or any combination thereof. Moreover, alteration of ATF2 activity provides first or second (or later) line approach to cancer therapy, and can be used alone or preferably in combination with a traditional therapeutic approach, *e.g.*, chemotherapy or radiation.

Peptide compounds that alter ATF2 activity or ATF2 vectors encoding the same, as described herein, can be formulated in a pharmaceutical composition for administration to a patient. As used herein, a "pharmaceutical composition" includes the active agent, *i*.*e*., the peptide, fusion protein or vector, and a pharmaceutically acceptable carrier, excipient, or diluent. The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal government or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water or oil, including those of petroleum, animal, vegetable or synthetic origin, such as peanut oil, soybean oil, mineral oil, sesame oil and the like. Water or aqueous solutions, saline solutions and aqueous dextrose and glycerol solutions are preferably employed as carriers, particularly for injectable solutions. Suitable pharmaceutical carriers are described in "Remington's Pharmaceutical Sciences" by E.W. Martin.

For human therapy, the pharmaceutical compositions, including each of the active agents, will be prepared in accordance with good manufacturing process (GMP) standards, as set by the Food & Drug Administration (FDA). Quality assurance (QA) and quality control (QC) standards will include testing for purity and function, in the case of polypeptides; homogeneity and function in the case of vectors; and the presence of replication competent virus (if the virus vector is defective) for viral vectors; and other standard measures.

In order to treat tumor cells, a pharmaceutical composition is administered by any route that will permit delivery of the active agent to a tumor cell. Since alteration of ATF2 activity does not appear to harm normal (non-transformed) cells, systemic administration of the active agent is acceptable. In certain embodiments, administration is parenteral, *e.g.*, via intravenous injection, or by other routes, such as intra-arteriole, intramuscular, intradermal, subcutaneous, intraperitoneal, intraventricular, and intracranial administration. Indeed, one of the advantages of this invention is that the specificity of the amino-terminal ATF2 peptides that alter ATF2 activity for transformed cells means that the active agent will effect metastatic cells, even micrometastases that cannot be resected or located by standard techniques (CAT scanning, MRI scanning, *etc*.). In other embodioments, delivery of a compound that alters ATF2 activity i.e. ATF2⁵¹⁻⁶⁰, or compositions thereof, is locally at the tumor, which can be topically or injection into a tumor mass.

In therapeutic treatments of the invention, the physician will administer a therapeutically effective amount of the pharmaceutical composition. As used herein, the term "therapeutically effective amount" means an amount sufficient to reduce by at least about 15 percent, preferably by at least 50 percent, more preferably by at least 90 percent, and most preferably prevent, a clinically significant deficit in the activity, function and response of the host. Alternatively, a therapeutically effective amount is sufficient to cause an improvement in a clinically significant condition in the host. Specifically, a therapeutically effective amount will cause one or more of the following: apoptosis of tumor cells; necrosis of tumor cells; elimination or prevention of tumor metastasises; reduction in the rate of tumor growth; reduction in tumor size or tumor shrinkage; elimination of the tumor; remission of the cancer; an increase in the time for reappearance of the cancer; and increased time of survival of the patient. The frequency and dosage of the therapy can be titrated by the ordinary physician using standard dose-to-response techniques.

### Combination Therapies

The therapeutic compositions of the invention can be used in combination with other anti-cancer strategies, as disclosed herein. In particular, as noted above, a particular advantage of altering ATF2 activity in accordance with the instant disclosure results from the synergistic effect of this strategy on traditional tumor therapies. Although the described methods are effective in inhibiting tumor growth and metastasis, the peptides, fusion proteins or vectors and the methods are advantageously used with other treatment modalities, including radiation and chemotherapy. In particular, compounds that alter ATF2 activity or nucleic acids that encode the same can be administered with a chemotherapeutic, such as a p38/JAK kinase inhibitor, *e.g.,* SB203580; a phospatidyl inositol-3 kinase (PI3K) inhibitor, *e.g*., LY294002; a MAPK inhibitor, *e.g.,* PD98059; a JAK inhibitor, *e.g.,* AG490; preferred chemotherapeutics such as UCN-01, NCS, mitomycin C (NMC), NCS, and anisomycin; taxanes such as taxol, taxotere and other taxoids (*e*.*g*., as disclosed in U.S. Patent Nos. 4,857,653; 4,814,470; 4,924,011, 5,290,957; 5,292,921; 5,438,072; 5,587,493; European Patent No. 0 253 738; and PCT Publication Nos. WO 91/17976, WO 93/00928, WO 93/00929, and WO 96/01815), or other chemotherapeutics, such as cis-platin (and other platin intercalating compounds), etoposide and etoposide phosphate, bleomycin, mitomycin C, CCNU, doxorubicin, daunorubicin, idarubicin, ifosfamide, vinca alkaloids, and the like.

The term "anti-tumor gene therapy" as used herein refers to a gene therapy targeted to a tumor, which causes or enhances tumor necrosis, apoptosis, growth regulation, *i*.*e*., regression or suppression of the tumor. Examples of anti-tumor gene therapies (in addition to delivery of an ATF2 peptide-encoding vector as set forth above) include, but are by no means limited to, introduction of a suicide gene; introduction of an apoptosis gene; introduction of a tumor suppresser gene; and introduction of an oncogene antagonist gene. Preferably anti-tumor genes are supplemented with immunostimulatory genes to enhance recruitment and activation of immune effector cells, including mobilized dendritic cells, to the tumor.

***Suicide gene therapies*.** Introduction of genes that encode enzymes capable of conferring to tumor cells sensitivity to chemotherapeutic agents (suicide gene) has proven to be an effective anti-tumor gene therapy. The present invention provides a method of treating cancer in part by introducing a gene vector, encoding a protein capable of enzymatically converting a prodrug, *i*.*e*., a non-toxic compound, into a toxic compound. In the method of the present invention, the therapeutic nucleic acid sequence is a nucleic acid coding for a product, wherein the product causes cell death by itself or in the presence of other drugs. A representative example of such a therapeutic nucleic acid is one which codes for thymidine kinase of herpes simplex virus. Additional examples are thymidine kinase of varicella zoster virus and the bacterial gene cytosine deaminase which can convert 5-fluorocytosine to the highly toxic compound 5-fluorouracil.

The prodrug useful in the methods of the present invention is any that can be converted to a toxic product, *i*.*e*., toxic to tumor cells. The prodrug is converted to a toxic product by the gene product of the therapeutic nucleic acid sequence in the vector useful in the method of the present invention. A representative example of such a prodrug is ganciclovir, which is converted *in vivo* to a toxic compound by HSV-tk. The ganciclovir derivative is toxic to tumor cells. Other representative examples of pro-drugs include acyclovir, FIAU [1-(2-deoxy-2-fluoro-beta-D-arabinofuranosyl)-5-iodouracil], 6-methoxypurine arabino-side for VZV-tk, and 5-fluorocytosine for cytosine deambinase.

Ganciclovir, or any of the pro-drugs, may be readily administered by a person having ordinary skill in this act. A person with ordinary skill would readily be able to determine the most appropriate dose and route for the administration of ganciclovir. Preferably, ganciclovir is administered in a dose of from about 1-20 mg/day/kg body weight. Preferably, acyclovir is administered in a dose of from about 1-100 mg/day/kg body weight and FIAU is administered in a dose of from about 1-50 mg/day/kg body weight.

HSV-tk based immunotherapy is built upon the fact that expression of the TK gene in conjunction with the drug ganciclovir (GCV) induces conditional toxicity in a transfected local tumor in addition to immune mediated inflammation (Chen et. al., Cancer Res. 1996, 56: 3758-3762).

***Anti-oncogene and tumor suppresser gene therapies*.** Tumor initiation and progression in many cancer types are linked to mutations in oncogenes (*e*.*g*., ras, myc) and tumor suppresser genes (*e.g.*, retinoblastoma protein, p53). A number of approaches are being pursued using anti-oncogene molecules, including monoclonal antibodies, single chain antibody vectors, antisense oligonucleotide constructs, ribozymes and immunogenic peptides (Chen, Mol. Med. Today 1997, 3:160-167; Spitz et al., Anticancer Res. 1996, 16:3415-3422; Indolfi et al., Nat. Med. 1996, 2:634-635; and Kijima et al., Pharmacol. Ther. 1995, 68:247-267). These molecules specifically inhibit the function of their target molecules, suppress tumor growth and increase the apoptosis rate in tumor cells. These mechanisms require constant presence of the suppresser or anti-oncogene molecules for sustained responses. However, these mechanisms by themselves have not been shown to induce tumor specific immunity, which has the potential of memory necessary for protection against the recurrence of the disease. Combination of these tumor growth specific strategies with DC mobilization will have a synergistic effect on tumor regression and induction of protective immune response.

Accordingly, in another embodiment, gene therapy for tumors includes but is by no means limited to p53 (PCT Publication No. WO 94/24297) or analogues thereof such as CTS-1 (French Patent Application No. FR 08729), anti-RAS single chain antibodies or antisense molecules (PCT Publication No. WO 97/16547), interferon-alpha or interferon-gamma, etc., as described above. Any vector for gene therapy can be used in conjunction with the present invention, such as a viral vector or naked DNA.

***Immunostimulatory therapies*.** The invention can provides for immune cell stimulation, such as dendritic cell mobilization, to generate a strong anti-tumor immune response. Immunostimulatory molecules include flt-3 ligand (flt-3L), granuclocyte-macrophage colony stimulating factor (GM-CSF), interleukin (IL)-12 and IL-13, IL-2, and IL-7. Other such cytokines include, but are not limited to, IL-3, and IL-4, a colony stimulating factor ("CSF") selected from the group consisting of granulocyte macrophage colony stimulating factor ("GM-CSF") or GM-CSF/IL-3 fusions, or other cytokines such as TNF-alpha or c-kit ligand.

Cytokines such as IL-12 amplify the antigen presenting and immunomodulatory capabilities of DC and inhibit tumor angiogenesis, which consecutively can induce immune susceptibility of the tumor. Conversely, cytokines such as IL-7 may induce more potent T cell responses and effectively reverse T cell defects *in vivo*. These cytokines can be administered as soluble or microparticle encapsulated protein or by introducing the gene encoding the cytokine in viral or non-viral vectors. Systemic delivery of such cytokines along with local anti-tumor gene therapies may increase the tumor distribution of these cytokines, which may be required for long term reversal of T cell defects and effective tumor responses. These cytokines, depending on the mode of administration, may have a critical role in exploiting the immune inflammation for an efficient anti-tumor immune response.

### Enhanced Combination Therapies

The present invention provides for further enhancement of the anti-tumor effect by including additional anti-tumor treatments with the compounds that alter ATF2 activity. For example, the present invention contemplates further combinations with tumor growth inhibitors, anti-angiogenesis treatment, tumor antigen and whole tumor vaccines, chemotherapeutic agents, radiation, and surgery (tumor resection).

***Tumor growth inhibitors*.** The term "tumor growth inhibitor" is used herein to refer to a protein that inhibits tumor growth, such as interferon (IFN)-γ, tumor necrosis factor (TNF)-α, TNF-β, and similar cytokines. Alternatively, a tumor growth inhibitor can be an antagonist of a tumor growth factor. Such antagonists include, but are not limited to, antagonists of tumor growth factor (TGF)-β and IL-10. The present invention contemplates administration of tumor growth inhibitor proteins systemically or, alternatively, by gene therapy.

***Anti-angiogenic factors*.** Tumor angiogenesis is an integral part of tumor progression and a variety of therapies targeted to inhibit angiogenesis are under development as cancer therapies. Anti-angiogenesis molecules vary from anti-angiogenic proteins *(e.g.,* angiostatin) to small molecules that block growth factor receptor mediated effects. Anti-angiogenesis therapies primarily reverse the growth/apoptosis balance of the tumor and induce dormancy. Once the administration of these therapies is halted, angiogenesis can resume and tumor growth progresses. Anti-angiogenesis is a powerful mechanism to specifically reduce the bulk of the tumor without adverse side effects in patients. The dormancy therapy induced by anti-angiogenesis paves the way for immunotherapy schemes to succeed by debulking the tumor, altering the tumor microenvironment, eliminating the immunosuppressive effects, and making the tumor more susceptible for immune mediated clearance.

An "anti-angiogenic factor" is a molecule that inhibits angiogenesis, particularly by blocking endothelial cell migration. Such factors include fragments of angiogenic proteins that are inhibitory (such as the ATF of urokinase), angiogenesis inhibitory factors, such as angiostatin and endostatin; soluble receptors of angiogenic factors, such as the urokinase receptor or FGF/VEGF receptor; molecules which block endothelial cell growth factor receptors (O'Reilly et. al., Cell 1997, 88:277-285; and O'Reilly, Nat. Med. 1996, 2:689-692), and Tie-1 or Tie-2 inhibitors. Generally, an anti-angiogenic factor for use in the invention is a protein or polypeptide, which may be encoded by a gene transfected into tumors using the vectors of the invention. For example, the vectors of the invention can be used to deliver a gene encoding an anti-angiogenic protein into a tumor in accordance with the invention. Examples of anti-angiogenic factors include, but are not limited to, the amino terminal fragment (ATF) of urokinase, containing the EGF-like domain (*e.g.*, amino acid residues about 1 to about 135 of ATF); ATF provided as a fusion protein, *e*.*g*., with immunoglobulin or human serum albumin (PCT Publication No. WO 93/15199); angiostatin (O'Reilly et al., Cell, 1994, 79:315-328); tissue inhibition of metalloproteinase (Johnson et al., J. Cell. Physiol., 1994, 160:194-202); or inhibitors of FGF or VEGF such as soluble forms of preceptors for angiogenic factors, including soluble VGF/VEGF receptor, and soluble urokinase receptor (Wilhem et al., FEBS Letters, 1994, 337:131-134). The present disclosure contemplates administration of anti-angiogenesis factors systemically or, alternatively, by gene therapy.

***Vaccines***. In order to increase the tumor antigen specific immune response, one could introduce defined tumor associated antigens (TAA) in the system to specifically increase the level of antigen. These TAA could be introduced as proteins, peptides or as genes in any viral or non-viral expression vectors. Immunization with these antigens could either follow or occur during anti-tumor therapy schemes. Essentially, this strategy enhances an effective immune response against specific antigen in conjunction with overall immune response. Specific immunization may lead to the expression of an immune enhancing cytokine, milieu which can promote the response against the antigens released by the tumor necrosis. Such immunization could be combined with immune activating cytokines (protein or genes) to further enhance the effects.

Besides the defined antigen based vaccines, a number of vaccine strategies are being explored in the laboratory as well as in the clinic. One well researched strategy in animal models is the modification of autologous or allogeneic tumor cell using cytokine genes (*e.g.*, IL-2, GM-CSF, IL-12, IL-4) as well as some key costimulatory molecule genes (*e.g.*, B7.1, B7.2). These gene modified tumor vaccines prove the concept of breaking peripheral tolerance and anergy using immunological mechanisms (Clary et al. Cancer Gene Ther., 1997, 4:97-104; and Gilboa, Semin. Oncol., 1996, 23:101-107). Other similar approaches include use of tumor lysates, proteins, or RNA pulsed DC and fusion of tumor cells with DC to induce a potent tumor immune response. All these approaches have a common theme, which is the delivery of antigenic molecules to the DC to induce efficient processing and presentation of these antigens to T cells.

### Screening and Chemistry

The recombinant cells of the invention that express a reporter gene under control of an ATF2-regulated expression control sequence, provide for development of screening assays, particularly for high throughput screening of molecules that up- or down-regulate the activity of the reporter gene expressed under the control of ATF2. Accordingly, the present invention contemplates methods for identifying specific antagonists and agonists of ATF2 that modulate its ability to regulate transciption using various screening assays known in the art. Such agonists and antagonists ("modulators") are referred to herein as "compounds". Compounds can be lead compounds for further development, or therapeutic candidates for pre-clinical and clinical testing.

Any screening technique known in the art can be used to screen for agonists or antagonists. The present invention contemplates screens for small molecules and mimics, as well as screens for natural products that bind to and agonize or antagonize ATF2-mediated transcription *in vivo.* For example, natural products libraries can be screened using assays of the invention for molecules that agonize or antagonize ATF2 transcription.

Knowledge of the primary sequence of the ATF2 inhibitory polypeptide fragment, and the similarity of that sequence with proteins of known function, can provide an initial clue as inhibitors or antagonists. Identification and screening of antagonists is further facilitated by determining structural features of the protein, *e.g.*, using X-ray crystallography, neutron diffraction, nuclear magnetic resonance spectrometry, and other techniques for structure determination. These techniques provide for the rational design or identification of agonists and antagonists.

The term "pharmacophore," as used herein, refers to a collection of functional groups (*e.g.*, atoms) on a protein or other compound of interest. More specifically, the term pharmacophore refers not only to the functional groups themselves, but also to their arrangement in three-dimensional space with respect to each other. In particular, the functional groups of a pharmacophore should be arranged in three-dimensional space with respect to each other in a manner that mimics or is substantially identical to their three-dimensional arrangement on the compound of interest. For example, the root-mean square deviation between functional groups in a compound of interest and in a pharmacophore should preferably be less than or equal to about one angstrom as calculated, *e.g.*, using the Molecular Similarity module within a molecular modeling program such as QUANTA (available from Molecular Simulations, Inc., San Diego, California). Preferred pharmacophores are derived from three-dimensional structures of the protein or other compound of interest that are experimentally determined, *e.g.*, by X-ray crystallography or by nuclear magnetic resonance (NMR) spectroscopy. However, suitable pharmacophores can also be derived, *e.g.*, from homology models based on the structures of related compounds or from three-dimensional structure-activity relationships. For example, preferred pharmacophores of the present invention are derived from the analysis of the interaction between ATF2, JNK and p38 polypeptides, or any combiniation thereof, and evaluating the effects of mutation in the residues that are involved of the interaction on ATF2 activity. Suitable pharmacophores can then be deduced or derived, *e.g.*, by correlating the effects of such mutations to three-dimensional, homology models of ATF2.

Pharmacophores of the present invention are particularly useful for identifying compounds, such as peptidomimetics, that modulate ATF2 activity in cells (either *in vitro* or *in vivo*). For example, in certain embodiments pharmacophores of the present disclosure can be used to identify compounds that compete with ATF2, *e.g.*, by binding to DNA, p38, JNK or any combination thereof, and inhibiting ATF2 from binding to DNA, p38, JNK or any combination thereof, but do not themselves generate any ATF2 activity. In certain embodiments, such compounds would effectively alter ATF2 activity, and preferably inhibit ATF2 activity (referred to as "antagonists" or "antagonist compounds").

Pharmacophores are generally more effective, and hence preferable, when they consist essentially of those unique functional groups or elements that are necessary for ATF2 activity, while having few, if any, functional groups or elements that do not affect such activity. Such pharmacophores would thereby simplify the search for ATF2 antagonists because the number of functional groups that must be compared between candidate compounds and the pharmacophore would be greatly reduced. Accordingly, the present invention provides, in preferred embodiments, an ATF2 pharmacophore that consists essentially of a designated number of functional groups or "pharmacophore points." Each of these points corresponds to a particular amino acid side chain in the ATF2 polypeptide sequence set forth in PubMed Accession No. NP 001871). More specifically, each point corresponds to a particular, unique atom or functional group on an amino acid side chain of that sequence. Accordingly, the pharmacophore points specify both the location of the amino acid residue, and a particular atom or functional group of that residue side chain.

As noted above, ATF2 pharmacophores of the present invention are particularly useful for identifying peptidomimetics and other compounds that are, for example, agonists or antagonists of ATF2 activity. Such compounds can be, for example, peptides and peptide analogues that comprise a portion of an ATF2 amino acid sequence (or an analogue thereof) corresponding to an ATF2 pharmacophore, *e.g.*, ATF2⁵¹⁻⁶⁰, ATF2⁵¹⁻⁷⁰, or ATF2⁵¹⁻⁸⁰, as disclosed herein. Alternatively, at least a portion of the peptidomimetics may be replaced by one or more non-peptide structures, such that the three-dimensional structure of functional groups in the pharmacophore is at least substantially retained. In other words, one, two, three or more amino acid residues within an ATF2 peptide may be replaced by a non-peptide structure. In addition, other portions of a peptide or peptidomimetic may be replaced by a non-peptide struccture.

Typically, peptidomimetics (both peptide and non-peptidyl analogues) may have improved properties (*e.g.*, decreased proteolysis, increased retention or increased bioavailability) that make them more suitable for pharmaceutical compositions than an ATF2 peptide. Peptidomimetics may also have improved oral availability. It should be noted that peptidomimetics of the instant disclosure may or may not have similar two-dimensional structures. However, all peptidomimetics will share common three-dimensional structural features and geometry. Each peptidomimetic of the invention may further have one or more unique additional binding elements. The present invention provides methods for identifying peptidomimetics, as described herein.

All peptidomimetics provided herein have a three-dimensional structure that is substantially similar to a three-dimensional structure of a pharmacophore as described above. Generally, the three-dimensional structure of a compound is considered substantially similar to that of a pharmacophore if the two structures have a root-mean square deviation (RMSD) less than or equal to about one angstrom, as calculated, *e.g.*, using the Molecular Similarity module with the QUANTA program (available from Molecular Simultaions, Inc., San Diego, California) or using other molecular modeling programs and algorithms that are available to those skilled in the art. In particular, a peptidomimetic of the invention will have at least one low-energy three-dimensional structure that is or is predicting to be (*e.g.,* by *ab-initio* modeling) substantially similar to the three-dimensional structure of a PF4 pharmacophore.

Lower energy conformations can be identified by conformational energy calculations using, for example, the CHARMM program (Brooks et al., J. Comput. Chem. 1983, 4:187-217). The energy terms include bonded and non-bonded terms, including bond length energy. It will be apparent that the conformational energy of a compound can also be calculated using any of a variety of other commercially available quantum mechanic or molecular mechanic programs. Generally, a low energy structure has a conformational energy that is within 50 kcal/mol of the global minimum.

As an example, and not by way of limitation, low energy conformations can be identified using combinations of two procedures. The first procedure involves a simulated annealing molecular dynamics approach. In this procedure, the system (which includes the designed peptidomimetics and water molecules) is heated up to above room temperature, preferably to around 600°Kelvin, and is simulated for a period of about 50 to 100 ps, or longer. Gradually, the temperature of the system is reduced, *e.g.*, to about 500 K and simulated for a period of about 100 ps or longer, then gradually reduced to 400 K and simulated for a period of 100 ps or longer. The system temperature is then reduced, again, to about 300 K and simulated for a period of about 500 ps or longer. During this analysis, the atom trajectories are recorded. Such simulated annealing procedures are well known in the art and are particularly advantageous, *e.g.*, for their ability to efficiently search the conformational "space" of a protein or other compound. That is to say, using such procedures it is possible to sample a large variety of possible conformations for a compound and rapidly identify those conformations having the lowest energy.

A second procedure involves the use of self-guided molecules dynamics (SGMD), as described by Wu & Wang, J. Physical Chem. 1998, 102:7238-7250. The SGMD method has been demonstrated to have an extremely enhanced conformational searching capability. Using the SGMD method, therefore, simulation may be performed at 300 K for 1000 ps or longer, and the atom trajectories recorded for analysis.

Conformational analysis ofpeptidomimetic and other compounds can also be carried out using the QUANTA molecular modeling package. First, cluster analysis may be performed using the trajectories generated from molecular dynamics simulations (as described herein). From each cluster, the lowest energy conformation may be selected as the representative conformation for this cluster and can be compared to other conformational clusters. Upon cluster analysis, major conformational clusters may be identified and compared to the solution conformations of the cyclic peptide(s). The conformational comparison may be carried out by using the Molecular Similarity module within the QUANTA program.

Similarity in structure can also be evaluated by visual comparison of the three-dimensional structures in graphical format, or by any of a variety of computational comparisons. For example, an atom equivalency may be defined in the peptidomimetic and pharmacophore three-dimensional structures, and a fitting operation used to establish the level of similarity. As used herein, an "atom equivanlency" is a set ofconserved atoms in the two structures. A "fitting operation" may be any process by which a candidate compound structure is translated and rotated to obtain an optimum fit with the cyclic peptide structure. A fitting operation may be a rigid fitting operation (*e*.*g*., the pharmacophore structure can be kept rigid and the three dimensional structure of the peptidomimetic can be translated and rotated to obtain an optimum fit with the pharmacophore structure). Alternatively, the fitting operation may use a least squares fitting algorithm that computes the optimum translation and rotation to be applied to the moving compound structure, such that the root mean square difference of the fit over the specified pairs of equivalent atoms is a minimum. Preferably, atom equivalencies may be established by the user and the fitting operation is performed using any of a variety of available software applications (*e.g.*, QUANTA, Molecular Simulations Inc., San Diego, California). Three-dimensional structures of candidate compounds for use in establishing substantial similarity can be determined experimentally (*e*.*g*., using NMR or X-ray crystallography techniques) or may be computer generated *ab initio* using, for example, methods provided herein.

As one example, chemical libraries (containing, *e.g.*, hydantoin and/or oxopiperazine compounds) may be made using combinatorial chemical techniques and initially screened, *in silico*, to identify compounds having elements of a PF4 pharmacophore of the invention, which are therefore likely to be either PF4 agonists or antagonists. Combinatorial chemical technology enables the parallel synthesis of organic compounds through the systematic addition of defined chemical components using highly reliable chemical reactions and robotic instrumentation. Large libraries of compounds result from the combination of all possible reactions that can be done at one site with all the possible reactions that can be done at a second, third or greater number of sites. Such methods have the potential to generate tens to hundreds of millions of new chemical compounds, either as mixtures attached to a solid support, or as individual, isolated compounds.

ATF2 pharmacophores can be used to greatly simplify and facilitate the screening of such chemical libraries to identify those compounds that are most likely to be effective antagonists of ATF2. As a result, library synthesis can focus on those library members with the greatest likelihood of interacting with the target (*e*.*g*., an ATF2 binding partner or the ATF2 polypeptide itself, and eliminate the need for synthesizing every possible member of a library (which often results in an unwieldy number of compounds). The integrated application of structure-based design and combinatorial chemical technologies can produce synergistic improvements in the efficiency of drug discovery. By way of example, hydantoin and oxopiperazine libraries may be limited to those compounds that involve only the addition of histidine and valine surrogates to the hydantoin or oxopiperazine backbone.

Peptidomimetic compounds of the present invention also include compounds that are or appear to be unrelated to the original ATF2 peptide, but contain functional groups positioned on a nonpeptide scaffold that serve as topographical mimics. Such peptiomimetics are referred to here as "non-peptidyl analogues." Non-peptidyl analogues can be identified, *e.g.*, using library screens of large chemical databases. Such screens use the three-dimensional conformation of a pharmacophore to search such databases in three-dimensional space. A single three-dimensional structure can be used as a pharmacophore model in such a search. Alternatively, a pharmacophore model may be generated by considering the crucial chemical structural features present within multiple three-dimensional structures.

Any of a variety of databases of three-dimensional structures can be used for such searches. A database of three-dimensional structure can also be prepared by generating three-dimensional structures of compounds, and storing the three-dimensional structures in the form of data storage material encoded with machine-readable data. The three-dimensional structures can be displayed on a machine capable of displaying a graphical three-dimensional representation and programmed with instructions for using the data. Within preferred embodiments, three-dimensional structures are supplied as a set of coordinates that define the three-dimensional structure.

Preferably, the three-dimensional (3D) database contains at least 100,000 compounds, with small, non-peptidyl molecules having relatively simple chemical structures particularly preferred. It is also important that the 3D coordinates of compounds in the database be accurately and correctly represented. The National Cancer Institute (NCI) 3D-database (Milne et al., J. Chem. Inf. Comput. Sci., 1994, 34:1219-1224) and the Available Chemicals DIrector (ACD; MDL Information Systems, San Leandro, California) are two exemplary databases that can be used to generate a database of three-dimensional structures, using molecular modeling methods such as those described, *supra*. For flexible molecules, which can have several low-energy conformations, it is desirable to store and search multiple conformations. The Chem-X program (Oxford Molecular Group PLC, Oxford, United Kingdom) is capable of searching thousands or even millions of conformations for a flexible compound. This capability of Chem-X provides a real advantage in dealing with compounds that can adopt multiple conformations. Using this approach, hundreds of millions of conformations can be searched in a 3D-pharmacophore searching process.

Typically, a pharmacophore search will involve at least three steps. The first of these is generation of a pharmacophore query. Such queries can be developed from an evaluation of distances in the three-dimensional structure of the pharmacophore. Using the pharmacophore query, a distance bit screening is preferably performed on a database to identify compounds that fulfill the required geometrical constraints. In other words, compounds that satisfy the specified critical pair-wise distances are identified. After a compound passes the distance bit screening step, the program should next check to determine whether the compound meets substructural requirements that can also be specified in the pharmacophore query. Once a compound passes the distance metric and sub-structural check, it is subjected to a conformational analysis. In particular, conformations of the compound are generated and evaluated with regard to geometric requirements specified in the pharmacophore query. Compounds that have at least one low energy conformation satisfying the geometric requirement can be considered "hits," and are candidate compounds for ATF2 antagonists.

Those skilled in the art will appreciate that a compound structure may be optimized, *e.g.*, using screens as provided herein. Within such screens, the effect of specific alterations of a candidate compound on three-dimensional structure may be evaluated, *e.g.,* to optimize three-dimensional similarity to an ATF2 pharmacophore. Such alterations include, for example, changes in hydrophobicity, steric bulk, electrostatic properties, size and bond angle. Biological testing of candidate agonists and antagonists identified by these methods is also preferably used to confirm their activity.

Once an active peptidomimetic has been identified, related analogues can also be identified, *e.g.*, by two-dimensional similarity searching. Such searching can be performed, for example, using the program ISIS Base (Molecular Design Limited). Two-dimensional similarity searching permits the identification of other available, closely related compounds which may be readily screened to optimize biological activity.

Another approach uses recombinant bacteriophage to produce large libraries. Using the "phage method" (Scott and Smith, Science 1990, 249:386-390; Cwirla et al., Proc. Natl. Acad Sci. USA 1990, 87:6378-6382; and Devlin et al., Science 1990, 49:404-406), very large libraries can be constructed (10⁶-10⁸ chemical entities). A second approach uses primarily chemical methods, of which the Geysen method (Geysen et al., Molec. Immunol. 1986,23:709-715; and Geysen et al. J. Immunologic Methods 1987, 102:259-274; and the method of Fodor et al. (Science 1991, 251:767-773) are examples. Furka et al. (14th International Congress of Biochemistry 1988, Volume #5, Abstract FR:013; Furka, Int. J. Peptide Protein Res. 1991, 37:487-493), Houghton (U.S. Patent No. 4,631,211) and Rutter et al. (U.S. Patent No. 5,010,175) describe methods to produce a mixture of peptides that can be tested as agonists or antagonists.

In another aspect, synthetic libraries (Needels et al., Proc. Natl. Acad Sci. USA 1993, 90:10700-4; Ohlmeyer et al., Proc. Natl. Acad Sci. USA 1993, 90:10922-10926; Lam et al., PCT Publication No. WO 92/00252; and Kocis et al., PCT Publication No. WO 9428028) and the like can be used to screen for compounds according to the present invention.

Test compounds are screened from large libraries of synthetic or natural compounds. Numerous means are currently used for random and directed synthesis of saccharide, peptide, and nucleic acid based compounds. Synthetic compound libraries are commercially available from Maybridge Chemical Co. (Trevillet, Cornwall, UK), Comgenex (Princeton, NJ), Brandon Associates (Merrimack, NH), and Microsource (New Milford, CT). A rare chemical library is available from Aldrich (Milwaukee, WI). Alternatively, libraries of natural compounds in the form of bacterial, fungal, plant and animal extracts are available from *e.g*. Pan Laboratories (Bothell, WA) or MycoSearch (NC), or are readily producible. Additionally, natural and synthetically produced libraries and compounds are readily modified through conventional chemical, physical, and biochemical means (Blondelle et al., TIBTech 1996, 14:60).

### In Vitro Screening Methods

According to the present invention, a recombinant ATF2-reporter gene promoter activity system is constructed. Candidate agents are added to *in vitro* cell cultures of host cells, prepared by known methods in the art, and the activity of the reporter gene is measured. Various *in vitro* systems can be used to analyze the effects of a new compound on reporter gene expression under control of ATF2. Preferably, each experiment is performed in triplicate at multiple different dilutions of compound.

Reporter genes for use in the invention encode detectable proteins, include, but are by no means limited to, chloramphenicol transferase (CAT), -galactosidase (-gal), luciferase, green fluorescent protein (GFP) and derivatives thereof, yellow fluorescent protein and derivatives thereof, alkaline phosphatase, other enzymes that can be adapted to produce a detectable product, and other gene products that can be detected, *e.g.*, immunologically (by immunoassay).

GFP has been modified to produce proteins that remain functional but have different fluorescent properties. Heim et al (U.S. Patent 5,625,048) modified GFP resulting in amino-acid changes which exhibited different excitation and emission spectra with visibly distinct colors and increased intensities of emission. Bjorn et al. (PCT Publication No. WO 96/23898) developed a new construct which encoded a modified GFP but also contained an enzyme recognition site. Bjorn et al (PCT Publication No. WO 97/11094) also developed new fluorescent proteins with increased intensity compared to the parent proteins. Hauswirth et al. (PCT Publication No. WO 97/26633) developed a GFP protein optimized to provide higher levels of expression in mammalian cells. Gaitanaris et al. (PCT Publication No. WO 97/42320) modified GFP resulting to increase the intensity of fluorescence, *e.g.*, by some twenty times greater than wild-type GFP, therefore increasing the sensitivity of detection. Cubitt et al. (PCT Publication No. WO 98/06737) developed modified GFP which could be easily distinguished from the already known green and blue fluorescent proteins. Evans et al. (PCT Publication No. WO 98/21355) developed new GFP mutants excitable with blue and white light.

The host cell screening system of the invention permits two kinds of assays: direct activation assays (agonist screen) and inhibition assays (antagonist screen). An agonist screen involves detecting changes in the level of expression of the reporter gene by the host cell contacted with a test compound; generally, reporter gene expression increases. If the reporter gene is expressed, the test compound has not affected ATF2 transcription activity; if the reporter gene expression increases, the test compound is a candidate for developing an ATF2 activator drug for use in conditions where inhibition of apoptosis is desirable.

An antagonist screen involves detecting expression of the reporter gene by the host cell when contacted with a test compound. If there is no change in expression of the reporter gene, the test compound is not an effective antagonist. If reporter gene expression is reduced or eliminated, the test compound has altered ATF2-mediated gene expression, and is thus a candidate for development of a cancer therapeutic.

The reporter gene assay system described here may be used in a high-throughput primary screen for agonists and antagonists, or it may be used as a secondary functional screen for candidate compounds identified by a different primary screen, *e.g.*, a binding assay screen that identifies compounds that modulate ATF2 transcription activity.

### High-Throughput Screen

Agents according to the invention may be identified by screening in high-throughput assays, including without limitation cell-based or cell-free assays. It will be appreciated by those skilled in the art that different types of assays can be used to detect different types of agents. Several methods of automated assays have been developed in recent years so as to permit screening of tens of thousands of compounds in a short period of time (*see, e.g.,* U.S. Patent Nos. 5,585,277,5,679,582, and 6,020,141). Such high-throughput screening methods are particularly preferred. Alternatively, simple reporter-gene based cell assays such as the one described here are also highly desirable. The use of high-throughput screening assays to test for agents is greatly facilitated by the availability of large amounts of purified polypeptides, as provided by the invention.

### In Vivo Screening Methods

Intact cells or whole animals expressing a gene encoding ATF2 can be used in screening methods to identify candidate drugs.

In one series of embodiments, a permanent cell line is established. Alternatively, cells are transiently programmed to express an *ATF2* gene by introduction of appropriate DNA or mRNA, *e.g.*, using the vector systems described above. In still another embodiment, cells (such as human tumor cells) that express ATF2 endogenously can be sued. Identification of candidate compounds can be achieved using any suitable assay, including without limitation (i) assays that measure selective binding of test compounds to ATF2 (ii) assays that measure the ability of a test compound to alter (*i*.*e*., inhibit or enhance) a measurable activity or function of ATF2 and (iii) assays that measure the ability of a compound to alter (*i*.*e*., inhibit or enhance) the transcriptional activity of sequences derived from the promoter (*i*.*e*., regulatory) regions the *ATF2* gene.

The present invention will be better understood by reference to the following Examples, which are provided by way of exemplification and not by way of limitation.

### EXAMPLES

### EXAMPLE 1

### CELL CULTURE AND DERIVATION OF STABLE CELL LINES

Human melanoma LU1205 cells were maintained in MCDB153/L15 medium (4:1) supplemented with 5% fetal bovine serum (FBS), L-glutamine and antibiotics. FEMX are late-phase melanoma-derived cells, which were maintained in RPMI-1640 supplemented with 5% FBS, L-glutamine and antibiotics. The mouse melanoma cell line SW1 was maintained in DMEM supplemented with 10% FBS; 293T human embryo kidney cells were grown in DMEM supplemented with 10% calf serum and antibiotics at 37°C with 5% CO2. SW1 clones that stably express the ATF2 peptides were selected in the presence of G418 (600µg/ml). Positive clones were selected following confirmation of expression by westerns, immunohistochemistry or RT-PCR.

### EXAMPLE 2

### ATF2⁵¹⁻¹⁰⁰ ATTENUATS GROWTH OF HUMAN MELANOMAS IN NUDE MICE

The ability of expressed ATF2⁵¹⁻¹⁰⁰ peptide to affect the tumorigenicity of human melanoma cells *in vivo* was examined. To this end, growth of LU1205 and FEMX human melanoma cells were monitored in nude mice in the presence or absence of treatment that induced apoptosis *in vitro.* LU1205, FEMX and SW-1 cells that express control or ATF2⁵¹⁻¹⁰⁰ peptide were trypsinized, resuspended in PBS and injected SC (1x10⁶) into 6-7 week old mice in the lower flank. When the tumor reached the size of about 50mm³, 10µM of SB203580 was injected into 4 areas of the LU1205 tumors every 4 days during a 2 week period. For FEMX tumors, UCN-01 (5mg/Kg) was fed by gavage, three times per week for a total of 2weeks. Tumor growth was monitored every two days. Tissue samples were fixed in formalin and embedded in paraffin. Hematoxylin and eosin (H&E), TUNEL staining was performed as previously described (Bhoumik et al., Clin. Cancer Res., 2001;2:331-342).

Expression of ATF2⁵¹⁻¹⁰⁰ reduced the growth rates of the two human melanoma tumors (Figure 1A, 1B). An additional decrease in growth of tumors that express ATF2⁵¹⁻¹⁰⁰ was found when they were treated with the pharmacological inhibitor of p38 or the chemotherapeutic drug UCN-01 (Figure 1A, 1B). TUNEL analysis revealed a marked increase in the degree of apoptosis in tumors expressing the ATF2⁵¹⁻¹⁰⁰ peptide upon their exposure to additional treatment (Figure 1C). These results indicate that expression of the ATF2⁵¹⁻¹⁰⁰ peptide suffices to slow the growth rate and to sensitize human melanoma tumors to apoptosis upon treatment.

### EXAMPLE 3

### DELIVERY OF HIV-TAT-ATF2⁵¹⁻¹⁰⁰ FUISON PROTEIN TO SW1 TUMORS INHIBITS GROWTH AND METASTASIS

The ability of delivering ATF2⁵¹⁻¹⁰⁰ peptide to affect melanoma growth *in vivo* was examined. To this end, the HIV-TAT system was chosen given its ability to elicit potent delivery of its fusion proteins through cellular membranes (Vocero-Akbani et al., Methods Enzymol., 2000, 322:508-21). The short ATF2 peptides were generated by introducing stop codons at the respective position into HA-penetratin pcDNA3 ATF2 51-100 amino acid vector (Bhoumik et al., Clin. Cancer Res., 2001; 2:331-342) using the Quick Change Site-Directed Mutagenesis Kit (Stratagene). DNA sequencing and RT-PCR were carried out in all cases to confirm the integrity of each construct as well as its expression levels. Amino acids 51-100 of ATF2 were cloned into the HIV-TAT construct (Vocero-Akbani et al., Methods Enzymol., 2000; 322:508-21) within the NcoI and XhoI sites. The fusion protein was expressed in *E. coli* BL21 (DE3) pLysS (Novagen, Madison, WI), and proteins were induced following the standard IPTG protocol before being subjected to purification with the aid of Ni-NTA beads.

SW-1 cells that express wt or mutant forms of the ATF2 peptide were trypsinized, resuspended in PBS and injected subcutaneously in the lower flank (1 x 10⁴) of 6-7 week old mice as previously described (Bhoumik et al., J. Clin. Invest., 2002; 110:643-650). GFP-expressing SW1 tumors were monitored *in vivo* on shaved mice at the indicated time points using a light box illuminated by blue light fiber optics (Lightools Research, Encinitas, CA) and imaging was carried out using a digital camera (Nikon D100). Injection of HIV-TAT, control or fused with ATF2 peptide, was performed on 48mm³ size tumors at the indicated time points. Each injection was directed at multiple sites within the tumors. The retroviral vector used to express GFP in SW1 cells is a derivative of the Moloney Murine Leukemia virus vector pMMP412 into which an internal ribosome entry site-puromycin resistance cassette was inserted downstream of the GFP.

Following HIV-TAT-ATF⁵¹⁻¹⁰⁰ peptide production in *E*. *coli* and purification on nickel beads (Figure 2A), 1 µg/mm³ of the peptide was injected into 48mm³ -size SW1 tumors followed by a second injection at the indicated time points (Figure 2B); thus, the amount of peptide injected was directly proportional to the size of the tumor. Prior to their injection into mice, tumor cells used in this study were infected with GFP, enabling follow up observations of tumor size in real time without need of surgery. Using a UV lamp allowed monitoring changes in tumor mass, as shown in Figure 2B. Whereas the control HIV-TAT construct had no effect on SW1 tumor growth, injection of the HIV-TAT-ATF2⁵¹⁻¹⁰⁰ fusion protein unexpectedly caused a marked decrease in tumor mass, which was noticeable within a day or two after the first injection and more so after 4 days (Figure 2B). These data provided the first evaluation of the ATF2 peptide's effect *in vivo* at different stages after its administration. Further analysis was carried out upon termination of the experiment, 13 days after the second injection. Of 20 tumors 9 were no longer visible after 3 days (2 tumors), 6 days (4 tumors), and 10 days (3 tumors) following the first injection of the HIV-TAT-ATF2⁵¹⁻¹⁰⁰ peptide. At the end of the experiment, 16 days after the first injection of the peptide, 11 of 20 tumors exhibited marked growth inhibition (Figure 2C).

Significantly, whereas the SW1 tumors injected with the control HIV-TAT peptide metastasized to the lungs to form multiple lesions, such tumors were no longer seen in any animal that received the HIV-TAT-ATF2⁵¹⁻¹⁰⁰ peptide (Figure 3A). Microscopic examination using H&E staining confirmed the presence of multiple metastatic lesions within the lungs of control animals but not in the ATF2⁵¹⁻¹⁰⁰ peptide-expressing group (Figure 3B). Lung metastases were significantly more pronounced upon visualization of GFP under a fluorescence microscope, which enabled detection of GFP-positive lesions (Figure 3C). These findings confirm that the GFP-expressing SW1 cells are those that metastasized to generate these lesions. Significantly, no such lesions were found in animals into which the HIV-TAT-ATF2⁵¹⁻¹⁰⁰ peptide had been injected (Figure 3C). Together, these data show that administration of the ATF2⁵¹⁻¹⁰⁰ peptide as an HIV-TAT fusion peptide into tumors unexpectedly results in efficient inhibition of tumorigenesis and metastasis of these otherwise aggressive tumors.

### EXAMPLE 4

### AMINO-ACIDS 51-60 OF ATF2 CAPABLE OF ALTERING ATF2 ACTIVITY

**Inhibition of Tumor Growth.** To examine the importance of the JNK association domain on ATF2, the possible role of a smaller peptide containing an intact JNK binding site (*i*.*e*., amino acids M⁵¹T⁵² of ATF2) was examined. To this end, three peptides of differing sizes (amino acids 51-80, 51-70, and 51-60) were compared to ATF2⁵¹⁻¹⁰⁰ peptide in their ability to inhibit tumor growth of SW1 cells *in vivo.* RT-PCR reactions confirmed the expression of these peptides' transcripts in the SW1 cells (Figure 4A). Subcutaneous injection of 10⁶ SW1 cells expressing the control construct resulted in formation of 1100 mm³ size tumors within 18 days. However, expression of the 50aa peptide in these cells (constitutive expression based on selection of drug-resistant cells) decreased tumor size to 300mm³. Strikingly, constitutive expression of each of the 3 shorter peptides also elicited inhibition of melanoma growth that varied from 400-600 mm³ (Figure 4B). Both the 20 amino acid and 10 amino acid peptides, spanning amino acids 51-60 and amino acids 51-70, respectively, elicited efficient inhibition close to that observed with the 50 amino acid peptide. These data indicate that the 51-60 amino acid peptide contains the domain required for inhibition of SW1 growth *in vivo.* Further evaluation was performed on this 10 amino acid peptide.

**Alteration of ATF2 Transcriptional Activity. Assessed next was** whether the short peptide caused any change in transcriptional activity mediated by ATF2 and its heterodimeric partners, using the TRE and Jun2 promoter sequences linked to the Luciferase marker gene. These reporter constructs (0.3 µg) were cotransfected with the respective expression vectors into melanoma cells (5x10⁵) using Lipofectamine (Invitrogen). Luciferase activity was measured using the Luciferase assay system (Promega).

Expression of ATF2⁵¹⁻¹⁰⁰ elicits a marked increase in the degree of TRE-Luc activity (Figure 5A). Similarly, expression of ATF2⁵¹⁻⁶⁰ was also efficient in increasing TRE-Luc activities (Figure 5A). These findings indicate that the short peptide elicits similar changes in promoters containing TRE sequences. In contrast, transcriptional activities from Jun2-Luc, which was efficiently inhibited upon expression of ATF2⁵¹⁻¹⁰⁰ (Figure 5B), were no longer inhibited and slightly elevated in response to expression of the 51-60 amino acid peptide. This indicates that inhibition of ATF2 transcriptional activities *via* the 50 amino acid peptide were no longer be mediated in response to expression of the shorter peptide. Surprisingly, these findings indicate that the 10aa peptide affects primarily the Jun/JNK signaling cascade *via* altered TRE-dependent activities.

**Effect on Apoptosis.** Assessed next was the degree of apoptosis in SW1 cells under normal growth (spontaneous) as well as following treatment with chemotherapeutic drugs. Cells were analyzed to detect spontaneous (basal) or induced apoptosis [following treatment with the kinase inhibitor UCN-01 (kindly provided by NCI repository) for 36 hours]. Apoptosis was measured by using FACS (Ivanov et al., Mol. Cell, 2001, 7:517-28) to quantify the percentage of hypodiploid nuclei undergoing DNA fragmentation. Nucleation as a marker of apoptosis was monitored via DAPI staining of cells at indicated time points after their treatment. Caspase activity was monitored by immunoblots using antibodies to caspase 9 (Santa Cruz). PARP cleavage was monitored by corresponding antibodies (PharMingen).

Normally, as is known in the art and as described herein, SW1 cells are highly resistant to apoptosis following various treatments. Such resistance was reduced upon expression of the ATF2⁵¹⁻⁶⁰ peptide, as treatment with the protein kinase inhibitor UCN-01 (shown as representative of various treatments) induced an increase (20-40%) in apoptosis (Figure 5C). However, the degree of UCN-01-induced apoptosis was substantially higher in melanoma cells that express the 50 amino acid peptide (20-77%; Figure 5C). However, SW1 cells that express the shorter peptide underwent spontaneous apoptosis, which was seen in the absence of treatment (12-37%; black bar, third panel, Figure 5C). Induction of basal apoptosis was also seen, at somewhat higher levels, upon expression of the ATF2⁵¹⁻¹⁰⁰ peptide (12-55%; black bar, second panel, Fig. 5c). These findings indicate that whereas sensitization by the full length peptide (aa 51-100) is mediated *via* two distinct mechanisms - spontaneous and inducible apoptosis - the shorter peptide (amino acids 51-60) primarily elicits spontaneous, and to a lesser extent, inducible apoptosis.

To assess which of the pro-apoptotic components is affected upon expression of ATF2 peptides, changes in the profile of caspases was analyzed. As shown in Figure 6, treatment of both human and mouse melanoma cells with UCN-01 caused activation of caspase 9, as reflected by the formation of the corresponding cleavage fragments. Cells that express either the 10 or 50 aa peptides revealed activation of caspase 9 even prior to treatment with UCN-01. Treatment with UCN-01 retained the same level of caspase 9 cleavage, as seen prior to treatment (Figure 6). These data indicate that the expression of the ATF2 peptides is sufficient for the activation of caspase 9, which could explain, in part, how expression of these peptides cause spontaneous apoptosis. Similarly, PARP cleavage was observed upon treatment of the control cells, but also in cells that express ATF2 peptides, prior to their exposure to UCN-01 (Figure 6). Indeed, caspase 3, which is responsible for PARP cleavage, was also activated upon expression of the ATF2 peptides (data not shown). These data indicate that both peptides suffice to induce caspase and PARP cleavage, which reflects cell commitment for apoptosis.

**Effect on JNK / Jun Association**. The short peptide's ability to elicit major changes in TRE-dependent transcription and spontaneous apoptosis led to an assessment of possible changes in the JNK-Jun association. Flag JNK2 expressed in 293T cells extracted, immunopurified with anti-Flag antibody bound to Protein G agarose beads were first incubated with 3mg/ml of BSA in phosphate-buffered saline for 2h, followed by incubation at 4°C for 2h with ³⁵S-labeled *in vitro* translated c-Jun or ATF2 (TNT-coupled reticulocyte lysate system, Promega) in the presence of WT or mutant peptide. Bead-bound material was subjected to three washes with 20mM Tris [pH 7.5], 150mM NaCl, 1mM EDTA, 1mM EGTA, 0.5% NP-40, 1mM NaVO₄, and 1mM DTT supplemented with protease inhibitors. Reaction mixtures were then separated on SDS-PAGE and transferred onto a nitrocellulose membrane. Binding was detected by autoradiography and quantified with the aid of a phosphorimager. The wild type and mutant ATF2⁵¹⁻⁶⁰ peptides were synthesized (Sigma Genosys) at a scale of 5mg with >95% purity.

The 51-100 amino acid ATF2 peptide binds to JNK, resulting in increased basal JNK activity (Bhoumik et al., Proc Nat'l. Acad Sci. USA, 2004, 101:4222-7) and thereby explaining the increase upon expression of this peptide in c-Jun's stability and activity (Bhoumik et al., J. Clin. Invest., 2002;110:643-650; Bhoumik et al., Proc Nat'l. Acad. Sci. USA, 2004, 101:4222-7). Similarly, the short peptide increases the *in vitro* association between JNK and c-Jun in a dose-dependent manner, also resulting in increased Jun transcriptional activities (Figures 7A-C). Such an effect was not observed in the JNK-ATF2 association (Figures 7A-C). This finding indicates that the ATF2⁵¹⁻¹⁰ peptide efficiently increases the affinity of JNK for c-Jun, enabling greater phosphorylation and activation, which coincide with higher stability.

### EXAMPLE 5

### GENE EXPRESSION PROFILING OF SW1 TUMORS EXPRESSING ATF2⁵¹⁻¹⁰⁰ PEPTIDE

To elucidate the molecular pathways affected by expression of the ATF2⁵¹⁻¹⁰⁰ peptide, we used cDNA microarray analysis to compare the RNA expression profiles of SW1 tumors that express this peptide or a control vector (Figure 8A). The 10k mouse Gem 2 gene set (Incyte Genomics Inc, Palo Alto, CA) was printed at the NCI on poly-L-lysine-coated glass using a Biorobotics TASII arrayer (Cambridge, England). All protocols for manufacturing and hybridization of microarrays are posted on the NCI web site (*see, e.g*., nciarray.nci.nih.gov). Approximately 20µg of total RNA were used in the reverse transcription reaction to directly label the probe with Cy-5 dUTP or Cy-3 dUTP (Amersham). Hybridizations were performed at 65°C for 12-18h in a hybridization volume of 35µL. The hybridized arrays were scanned using an Axon GenePix 4000 scanner (Union City, CA) and fluorescence data were collected using the GenePix software.

mRNA was prepared from a pool of 4 tumors obtained from two different experiments. In both cases, tumors were excised 2 weeks after subcutaneous injection of cells. Because the fluorescently labeled Cy dyes may incorporate into the reverse-transcribed cDNA with different efficiencies, RNA isolated from both cell types was always labeled with both combinations of Cy dyes. Genes either up- or down-regulated by more than 2.5 fold were identified on the basis of a scatter plot analysis (Figure 8A). SW1 vector control cells were labeled with Cy-3 (F532) and the SW1-ATF 51-100 cells were labeled with Cy-5 (F635). Genes that are upregulated in response to the expression of the ATF2⁵¹⁻¹⁰⁰ peptide appear red on the microarray whereas genes down regulated in tumors expressing the peptide (compared to control tumors) appear green. Clustering analysis of the array data revealed several interesting patterns of gene expression. A large group of tumor necrosis factor-related genes were strongly upregulated in tumors that express the ATF2⁵¹⁻¹⁰⁰ peptide (Figure 8B).

In addition, tumor suppressor genes and tumor rejection antigens were clustered within this group. Of interest, and in accordance with our results in the current study, we observed a down-regulation of Fas-associated genes in the tumors that express the ATF2⁵¹⁻¹⁰⁰ peptide (Figure 8B). Down-regulation of Fas-associated genes would result in reduced apoptosis via the Fas pathway and explain why there was no change in the growth and development of these tumors in *GLD* mice, which are deficient in Fas ligand (Bhoumik et al., J. Clin. Invest., 2002, 110:643-650, data not shown). Concomitant with decrease in Fas-associated genes was up-regulation of TNF-related transcripts, which constitutes the other major apoptosis cascade. These changes reveal that expression of the ATF2 peptide efficiently altered the balance of apoptosis cascades from Fas towards the TNF pathway, which otherwise provides a pro-mitogenic signal in late stage melanomas (Ivanov and Ronai, J. Biol. Chem., 1999, 274:14079-14089). Interestingly, we also observed a consistent down-regulation of growth-associated genes including epidermal growth factor, hepatoma-derived growth factor and insulin-like growth factor, and several interferon-associated genes (Figure 8B).

Among other up-regulated transcripts (Table 1A) were insulin-like growth factor binding protein 2 (implicated in IGF activity, which has been associated with radio-resistance and apoptosis; Macaulay et al., Oncogene, 2001, 20:4029-40; Kanter-Lewensohn et al., Melanama Res., 1998, 8:389-97), interleukin 1 beta (implicated in inhibition of angiogenesis and metastasis of melanoma and other tumors; Belardelli et al., Int. J. Cancer, 1989; 44:1108-16), cullin 3 (ubiquitin protein ligase that controls cyclin E in ubiquitination and consequently regulates entry into the S phase; Singer et al., Genes Dev., 1999; 13:2375-87; Winston et al., Genes Dev., 1999; 13:2751-7), kinesin-associated protein 3 (a kinesin superfamily-associated protein implicated in organelle transport; Yamazaki et al., Proc. Nat'l. Acad. Sci. USA, 1996; 93:8443-8; Manning and Snyder, Trends Cell. Biol., 2000, 10:281-9), ATF3 (which represses cyclic-AMP responsive element [CRE]-dependent transcription and accelerates caspase protease activation during DNA damaging agent-induced apoptosis; Mashima et al., J. Cell. Physiol., 2001, 188:352-8) and membrane metallo-endopeptidase (implicated in invasion and metastasis; Hofmann et al., J. Invest. Dermatol., 2000, 115:337-44).

Among the additional transcripts down-regulated in ATF2-expressing tumors (Table 1B) were tyrosine kinase 2, plasminogen activator (implicated in invasion and metastasis; Duffy, Curr. Pharm. Des., 2004, 10:39-49), metallothionein 2 (which confers resistance to metals; Czaja et al., J. Cell. Physiol., 1991, 147:434-8), microtubule-associated protein myosin Vb (associated with the plasma membrane recycling system; Lapierre et al., Mol. Biol. Cell, 2001;12:1843-57), and ubiquitin-specific protease 18 (a type I interferon-inducible gene that contributes to growth arrest and differentiation in human melanoma cells treated with IFN-beta; Kang et al., Gene, 2001, 267:233-42). This panel provides important mechanistic insights with regard to changes in the expression pattern of genes that occurred in vivo in the course of inhibiting tumor growth in response to expression of the ATF2⁵¹⁻¹⁰⁰ peptide.

The present invention is not to be limited in scope by the specific embodiments described herein. Indeed, various modifications of the invention in addition to those described herein will become apparent to those skilled in the art from the foregoing description and the accompanying figures. It should be further understood that all values are approximate, and are provided for description.

**Table 1A: Ratios of genes upregulated in 4 independent hybridizations. ATFp2-Cy 5 vs parental control-Cy3**

| **#1** | **#2** | **#3** | **#4** | **Var.** | **Feature ID** | **Gene** | **Description** |
|---|---|---|---|---|---|---|---|
| 6.2407 | 6.2633 | 5.4673 | 4.7632 | 0.026 | IMAGE:776163 | C85356 | expressed sequence C85356 |
| 5.0430 | 5.0612 | 4.9219 | 4.6123 | 0.036 | IMAGE:1068748 | Tna | tetranectin (plasminogen-binding protein) |
| 4.8477 | 4.8652 | 4.8096 | 4.5247 | 0.002 | IMAGE:849871 | Mme | membrane metallo endopeptidase |
| 3.6935 | 3.7069 | 4.4104 | 4.1619 | 0.012 | IMAGE:596683 | Scyb13 | small inducible cytokine subfamily B (Cys-X-Cys), member 13 |
| 3.6319 | 3.6450 | 3.4859 | 3.4284 | 0.001 | IMAGE: 1052178 | Akrlc13 | aldo-keto reductase family 1 member C13 |
| 3.0625 | 3.0736 | 4.2117 | 3.5432 | 0.035 | IMAGE:535754 | D0H4S114 | DNA segment, human D4S114 |
| 3.0715 | 3.0826 | 3.4986 | 4.0870 | 0.028 | IAMGE:1277614 | lgj | immunoglobulin joining chain |
| 2.3638 | 2.3724 | 4.2802 | 5.0410 | 0.243 | IMAGE:961070 | Cul3 | cullin 3 |
| 2.7162 | 2.7260 | 3.6427 | 3.9752 | 0.061 | IMAGE:960604 | | RIKEN cDNA 1110068L01 gene |
| 3.2649 | 3.2767 | 3.3268 | 3.0043 | 0.003 | IMAGE:1277469 | lgfbp2 | insulin-like growth factor binding protein 2 |
| 3.1997 | 3.2112 | 2.7725 | 3.6146 | 0.018 | IMAGE:332935 | Hbb-b1 | hemoglobin beta adult major chain |
| 2.6799 | 2.6896 | 3.4635 | 3.3665 | 0.03 | IMAGE:1107479 | Top1 | topoisomerase (DNA) 1 |
| 2.0425 | 2.0498 | 4.1525 | 4.7416 | 0.316 | IMAGE:1210910 | Slap | sarcolemmal-associated protein |
| 2.5423 | 2.5514 | 3.4806 | 3.3033 | 0.043 | IMAGE: 1139544 | ll1b | interleukin 1 beta |
| 2.3816 | 2.3902 | 3.1712 | 3.6075 | 0.068 | IMAGE:1399769 | Sp3 | trans-acting transcription factor 3 |
| 2.8994 | 2.9099 | 2.4766 | 2.5621 | 0.011 | IMAGE:518979 | Hbb-b 1 | hemoglobin, beta adult major chain |
| 2.1192 | 2.1268 | 2.9811 | 3.9220 | 0.138 | IMAGE:331395 | Kifap3 | kinesin-associated protein 3 |
| 2.5956 | 2.6050 | 2.6414 | 2.8732 | 0.004 | IMAGE:2101997 | Lum | lumican |
| 2.7606 | 2.7705 | 2.7398 | 2.3263 | 0.011 | IMAGE:902951 | Ogn | osteoglycin |
| 2.6457 | 2.6552 | 2.6858 | 2.3889 | 0.005 | IMAGE:809168 | | ESTs |
| 2.6354 | 2.6449 | 2.6270 | 2.3980 | 0.003 | IMAGE:1480170 | Efemp1 | epidermal growth factor-containing fibulin |

**Table 1B: Ratio of genes down regulated in 4 independent hybridizations ATFp2-Cy5 vs parental control-Cy3**

| **#1** | **#2** | **#3** | **#4** | **Var.** | **Feature ID** | **Gene** | **Description** |
|---|---|---|---|---|---|---|---|
| 0.1598 | 0.1572 | 0.1540 | 0.1537 | 0.001 | IMAGE:1022928 | Xir | X-linked lymphocyte-regulated complex |
| 0.2354 | 0.2363 | 0.2637 | 0.3054 | 0.023 | IMAGE:616653 | lfit1 | interferon-induced protein with tetratricopeptide repeats 1 |
| 0.2748 | 0.2758 | 0.2551 | 0.2439 | 0.006 | IMAGE:1378935 | Myo5b | Myosin Vb |
| 0.3227 | 0.3239 | 0.2740 | 0.3014 | 0.01 | IMAGE:516935 | | RIKEN cDNA 2900002K07 gene |
| 0.3213 | 0.3225 | 0.2989 | 0.2958 | 0.003 | IMAGE:751642 | Usp18 | ubiquitin specific protease 18 |
| 0.2911 | 0.2922 | 0.3318 | 0.4279 | 0.051 | IMAGE:620800 | Scya12 | Small inducible cytokine A12 |
| 0.3248 | 0.361 | 0.3150 | 0.3569 | 0.008 | IMAGE:406272 | Crabp1 | cellular retinoic acid binding protein 1 |
| 0.3486 | 0.3498 | 0.362 | 0.3547 | 0.008 | IMAGE:720190 | S100a9 | S100 calcium-binding protein A9 (calgranulin B) |
| 0.4938 | 0.4955 | 0.3268 | 0.3091 | 0.103 | IMAGE:621129 | | ESTs |
| 0.3803 | 0.3817 | 0.3913 | 0.4599 | 0.013 | IMAGE:577544 | Ptk2 | PTK2 protein tyrosine kinase 2 |
| 0.4750 | 0.4768 | 0.3824 | 0.3361 | 0.046 | IMAGE:1210036 | lfit3 | interferon-induced protein with tetratricopeptide repeats 3 |
| 0.4421 | 0.4583 | 0.383 | 0.3949 | 0.012 | IMAGE:864447 | G0s2 | G0/G1 switch gene 2 |
| 0.4074 | 0.4089 | 0.4019 | 0.5488 | 0.036 | IMAGE:836124 | Plat | plasminogen activator, tissue |
| 0.3565 | 0.3578 | 0.4847 | 0.6503 | 0.129 | IMAGE:636695 | lgh-1 | Immunoglobulin heavy chain 1 (serum lgG2a) |
| 0.3874 | 0.3888 | 0.4649 | 0.5780 | 0.056 | IMAGE:334351 | Mt2 | metallothionein 2 |
| 0.4381 | 0.4397 | 0.4481 | 0.4781 | 0.003 | IMAGE:1023899 | Lgals7 | Lectin galactose binding soluble 7 |
| 0.4501 | 0.4517 | 0.4544 | 0.4479 | 0 | IMAGE:1278511 | Ensa | endosulfine alpha |
| 0.3777 | 0.3791 | 0.4947 | 0.6368 | 0.097 | IMAGE:636228 | Casp6 | caspase 6 |
| 0.4768 | 0.4785 | 0.4402 | 0.4792 | 0.003 | IMAGE:1037652 | | mus musculus, clone MGC:19199 IMAGE:4236882, mRNA, complete cds |
| 0.3738 | 0.3752 | 0.5471 | 0.6286 | 0.11 | IMAGE:368571 | Hadh | hydroxylacyl-Coenzyme A dehydrogenase |
| 0.3697 | 0.3710 | 0.5261 | 0.7243 | 0.162 | IMAGE: 1430217 | Mtap6 | microtubule-associated protein 6 |
| 0.4707 | 0.4724 | 0.3928 | 0.6046 | 0.049 | IMAGE:619632 | | ESTs |
| 0.321 | 0.4337 | 0.4643 | 0.5141 | 0.005 | IMAGE: 1068714 | lsg15 | interferon-stimulated protein (15 kDa) |

## Claims

1. An amino-terminal peptide of ATF2 consisting of amino acids from residue 51 of ATF2 to 60 of ATF2 for use as a pharmaceutical composition.

2. An amino-terminal peptide of ATF2 consisting of amino acids from residue 51 of ATF2 to 60 of ATF2 for use as a pharmaceutical composition for treatment of a cancer in which tumor cells demonstrate resistance to apoptosis, radiation, chemotherapeutic agents, or any combination thereof.

3. Use of an amino-terminal peptide of ATF2 consisting of amino acids from residue 51 of ATF2 to 60 of ATF2 for the manufacture of a pharmaceutical composition for treatment of a tumor cell comprising contacting the tumor cell with said peptide that alters ATF2 activity.

4. Use according to claim 3 where amino-terminal peptide inhibits growth of a tumor.

5. Use according to claim 3 where the amino-terminal peptide is sensitizing a tumor to apoptosis.

6. Use according to any one of claims 3 to 5, wherein the ATF2 peptide alters TRE-dependent transcriptional activity.

7. Use according to any one of claims 3 to 5, wherein the ATF2 peptide alters the association of JNK and c-Jun.

8. Use according to any one of claims 3 to 5, wherein the ATF2 peptide induces spontaneous apoptosis.

9. Use according to claim 3 wherein the tumor cell is a melanoma cell.

10. Use according to claim 3 wherein the tumor cell is a breast cancer cell.

11. Use according to claim 3, further comprising treating the tumor cell with a chemotherapeutic agent.

12. Use according to claim 11 wherein the chemotherapeutic agent is selected from the group consisting of a p38 inhibitor, UCN-01, NCS, anisomycin, LY294002, PD98059, AG490, and SB203580.

13. Use according to claim 3, further comprising treating the tumor cell with radiation.

14. Use according to any one of claims 3 to 5 wherein the ATF2 peptide further comprises a translocation peptide sequence.

15. Use according to claim 14 wherein the translocation peptide sequence is penetratin or HIV-TAT.

16. A peptide comprising an amino-terminal peptide fragment of ATF2, wherein said ATF2 peptide consists of amino acids from residue 51 of ATF2 to 60 of ATF2.

17. The peptide of claim 16, further comprising a translocation peptide sequence.

18. The peptide of claim 17 wherein the translocation peptide sequence is penetratin or HIV-TAT.

19. A nucleic acid encoding an ATF2 peptide according to claim 16.

20. An expression vector comprising the nucleic acid of claim 19 operably associated with an expression control sequence.

21. The expression vector of claim 20, wherein the expression control sequence provides for expression in a tumor cell.

22. Pharmaceutical composition comprising an amino-terminal peptide of claim 16 consisting of amino acids from residue 51 of ATF2 to 60 of ATF2 and a pharmaceutically acceptable carrier or excipient.

## Patentansprüche

1. Aminoterminales Peptid von ATF2, bestehend aus Aminosäuren von Rest 51 von ATF2 bis 60 von ATF2, zur Verwendung als eine pharmazeutische Zusammensetzung.

2. Aminoterminales Peptid von ATF2, bestehend aus Aminosäuren von Rest 51 von ATF2 bis 60 von ATF2, zur Verwendung als eine pharmazeutische Zusammensetzung zur Behandlung eines Krebses, in welchem Tumorzellen eine Resistenz gegen Apoptose, Strahlung, Chemotherapeutika oder eine beliebige Kombination davon aufweisen.

3. Verwendung eines aminoterminalen Peptids von ATF2, bestehend aus Aminosäuren von Rest 51 von ATF2 bis 60 von ATF2, für die Herstellung einer pharmazeutischen Zusammensetzung für die Behandlung einer Tumorzelle, umfassend das Inkontaktbringen der Tumorzelle mit dem Peptid, welches die ATF2-Aktivität ändert.

4. Verwendung nach Anspruch 3, wobei das aminoterminale Peptid das Wachstum eines Tumors hemmt.

5. Verwendung nach Anspruch 3, wobei das aminoterminale Peptid einen Tumor für die Apoptose sensibilisiert.

6. Verwendung nach einem der Ansprüche 3 bis 5, wobei das ATF2-Peptid die TREabhängige transkriptionelle Aktivität ändert.

7. Verwendung nach einem der Ansprüche 3 bis 5, wobei das ATF2-Peptid die Assoziation von JNK und c-Jun ändert.

8. Verwendung nach einem der Ansprüche 3 bis 5, wobei das ATF2-Peptid spontane Apoptose induziert.

9. Verwendung nach Anspruch 3, wobei die Tumorzelle eine Melanomzelle ist.

10. Verwendung nach Anspruch 3, wobei die Tumorzelle eine Brustkrebszelle ist.

11. Verwendung nach Anspruch 3, außerdem umfassend das Behandeln der Tumorzelle mit einem Chemotherapeutikum.

12. Verwendung nach Anspruch 11, wobei das Chemotherapeutikum ausgewählt ist aus der Gruppe bestehend aus einem p38-Inhibitor, UCN-01, NCS, Anisomycin, LY294002, PD98059, AG490 und SB203580.

13. Verwendung nach Anspruch 3, außerdem umfassend das Behandeln der Tumorzelle mit Strahlung.

14. Verwendung nach einem der Ansprüche 3 bis 5, wobei das ATF2-Peptid außerdem eine Translokationspeptidsequenz umfasst.

15. Verwendung nach Anspruch 14, wobei die Translokationspeptidsequenz Penetratin oder HIV-TAT ist.

16. Peptid, umfassend ein aminoterminales Peptidfragment von ATF2, wobei das ATF2-Peptid aus Aminosäuren von Rest 51 von ATF2 bis 60 von ATF2 besteht.

17. Peptid nach Anspruch 16, außerdem umfassend eine Translokationspeptidsequenz.

18. Peptid nach Anspruch 17, wobei die Translokationspeptidsequenz Penetratin oder HIV-TAT ist.

19. Nukleinsäure, codierend ein ATF2-Peptid nach Anspruch 16.

20. Expressionsvektor, umfassend die Nukleinsäure nach Anspruch 19 funktionsfähig assoziiert mit einer Expressionskontrollsequenz.

21. Expressionsvektor nach Anspruch 20, wobei die Expressionskontrollsequenz für eine Expression in einer Tumorzelle sorgt.

22. Pharmazeutische Zusammensetzung, umfassend ein aminoterminales Peptid nach Anspruch 16, bestehend aus Aminosäuren von Rest 51 von ATF2 bis 60 von ATF2, und einen pharmazeutisch verträglichen Träger oder Exzipienten.

## Revendications

1. Peptide amino-terminal d'ATF2 constitué des acides aminés du résidu 51 d'ATF2 au résidu 60 d'ATF2 pour une utilisation en tant que composition pharmaceutique.

2. Peptide amino-terminal d'ATF2 constitué des acides aminés du résidu 51 d'ATF2 au résidu 60 d'ATF2 pour une utilisation en tant que composition pharmaceutique pour le traitement d'un cancer dans lequel les cellules tumorales montrent une résistance à l'apoptose, aux radiations, aux agents chimiothérapeutiques, ou toute combinaison de ceux-ci.

3. Utilisation d'un peptide amino-terminal d'ATF2 constitué des acides aminés du résidu 51 d'ATF2 au résidu 60 d'ATF2 pour la fabrication d'une composition pharmaceutique pour le traitement d'une cellule tumorale comprenant la mise en contact de la cellule tumorale avec ledit peptide qui altère l'activité d'ATF2.

4. Utilisation selon la revendication 3, où le peptide amino-terminal inhibe la croissance d'une tumeur.

5. Utilisation selon la revendication 3, où le peptide amino-terminal sensibilise une tumeur à l'apoptose.

6. Utilisation selon l'une quelconque des revendications 3 à 5, dans laquelle le peptide ATF2 altère l'activité transcriptionnelle dépendante de TRE.

7. Utilisation selon l'une quelconque des revendications 3 à 5, dans laquelle le peptide ATF2 altère l'association de JNK à c-Jun.

8. Utilisation selon l'une quelconque des revendications 3 à 5, dans laquelle le peptide ATF2 induit une apoptose spontanée.

9. Utilisation selon la revendication 3, dans laquelle la cellule tumorale est une cellule de mélanome.

10. Utilisation selon la revendication 3, dans laquelle la cellule tumorale est une cellule de cancer du sein.

11. Utilisation selon la revendication 3, comprenant en outre le traitement de la cellule tumorale avec un agent chimiothérapeutique.

12. Utilisation selon la revendication 11, dans laquelle l'agent chimiothérapeutique est choisi dans le groupe constitué d'un inhibiteur de p38, UCN-01, NCS, anisomycine, LY294002, PD98059, AG490,et SB203580.

13. Utilisation selon la revendication 3, comprenant en outre le traitement de la cellule tumorale par radiation.

14. Utilisation selon l'une quelconque des revendications 3 à 5, dans laquelle le peptide ATF2 comprend en outre une séquence de translocation de peptide.

15. Utilisation selon la revendication 14, dans laquelle la séquence de translocation de peptide est la pénétratine ou HIV-TAT.

16. Peptide comprenant un fragment du peptide amino-terminal d'ATF2, dans lequel ledit peptide ATF2 est constitué des acides aminés du résidu 51 d'ATF2 au résidu 60 d'ATF2.

17. Peptide selon la revendication 16, comprenant en outre une séquence de translocation de peptide.

18. Peptide selon la revendication 17, dans lequel la séquence de translocation de peptide est la pénétratine ou HIV-TAT.

19. Acide nucléique codant pour un peptide ATF2 selon la revendication 16.

20. Vecteur d'expression comprenant l'acide nucléique selon la revendication 19 lié de manière opérationnelle à une séquence de contrôle de l'expression.

21. Vecteur d'expression selon la revendication 20, dans lequel la séquence de contrôle de l'expression permet l'expression dans une cellule tumorale.

22. Composition pharmaceutique comprenant un peptide amino-terminal selon la revendication 16 constitué des acides aminés du résidu 51 d'ATF2 au résidu 60 d'ATF2 et un support ou excipient pharmaceutiquement acceptable.
